# EUROPÄISCHE PATENTSCHRIFT

(11) **EP 0 422 469 B1**
(45) Veröffentlichungstag und Bekanntmachung des Hinweises auf die Patenterteilung: **08.05.1996**
(21) Anmeldenummer: 90118750.0
(22) Anmeldetag: 29.09.1990
(51) Int. Cl.: C07D 249/12, A01N 43/653, C07D 401/12, C07D 409/12, C07D 403/04

(54) **Sulfonylaminocarbonyltriazolinone**
Sulfonylaminocarbonyltriazolinones
Sulfonylaminocarbonyltriazolinones

(30) Priorität: 12.10.1989 DE 3934081
(43) Veröffentlichungstag der Anmeldung: 17.04.1991
(60) Teilanmeldung: 95111736.5
(73) Patentinhaber: BAYER AG, D-51368 Leverkusen (DE)
(72) Erfinder: Müller, Klaus-Helmut, Dr., D-4000 Düsseldorf 13 (DE); Babczinski, Peter, Dr., D-5600 Wuppertal 1 (DE); Santel, Hans-Joachim, Dr., D-5090 Leverkusen 1 (DE); Schmidt, Robert R., Dr., D-5060 Bergisch Gladbach 2 (DE)

(56) Entgegenhaltungen:
- EP-A- 0 283 876
- EP-A- 0 332 133
- EP-A- 0 341 489
- BE-A- 894 856
- FR-A- 2 551 439
- CHEMICAL ABSTRACTS, Band 11, 1990, Seite 714, Spalte 138972f, Columbus Ohio,US; A. IKIZLER et al.: "Preparation of some 1,2,4-triazolin-5-one derivatives",& DOGA: TURK KIM. DERG. 1988,12(3), 271-5
- CHEMICAL ABSTRACTS, Band 57, 1962, Spalte 2229e, Columbus, Ohio, US;& DE-A-1 126 882 (KARL-HEINZ HAUPTMANN AND KARL ZEILE) 05-04-1962

## Beschreibung

Die Erfindung betrifft neue Sulfonylaminocarbonyltriazolinone, mehrere Verfahren zu ihrer Herstellung und ihre Verwendung als Herbizide.

Es ist bekannt, daß bestimmte substituierte Aminocarbonylimidazolidinone, wie z. B. 1-Isobutylaminocarbonyl-2-imidazolidinon (Isocarbamid), herbizide Eigenschaften aufweisen (vgl. R. Wegler, Chemie der Pflanzenschutz- und Schädlingsbekämpfungsmittel` Band 5, S. 219, Springer-Verlag, Berlin-Heidelberg-New York, 1977). Die Wirkung dieser Verbindung ist jedoch nicht in allen Belangen zufriedenstellend.

Es wurden nun die neuen Sulfonylaminocarbonyl-triazolinone der allgemeinen Formel (I), 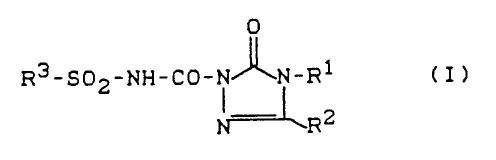
in welcher
- R¹: für Allyl, für C₃-C₆-Cycloalkyl, für Phenyl, für Benzyl, für C₁-C₃-Alkoxy, für C₁-C₃-Alkylamino oder für Di-(C₁-C₂-alkyl)-amino steht,
- R²: für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
- R³: für die Gruppierung 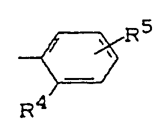 steht, worin
- R⁴: für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
- R⁵: für Wasserstoff, Fluor, Chlor oder Brom steht;
weiterhin
- R³: für den Rest 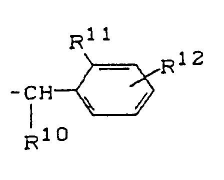 steht, worin
- R¹⁰: für Wasserstoff steht,
- R¹¹: für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
- R¹²: für Wasserstoff steht;
weiterhin
- R³: für den Rest 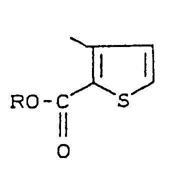 steht,
worin R für C₁-C₄-Alkyl steht, oder
für den Rest 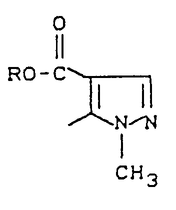 steht,
worin R für C₁-C₄-Alkyl steht,
sowie Salze von Verbindungen der Formel (I) gefunden,
ausgenommen jedoch - für die Vertragsstaaten BE, CH, DE, FR, GB, IT, LI und NL - die Verbindungen:
4-Cyclopropyl-5-methyl-2-(2-methoxycarbonyl-phenylsulfonyl-aminocarbonyl)-2,4-dihydro-3H-1,2,4-triazol-3-on
und
4-Dimethylamino-5-methyl-2-(2-methoxycarbonyl-phenylsulfonyl-aminocarbonyl)-2,4-dihydro-3H-1,2,4-triazol-3-on.

Man erhält die neuen Sulfonylaminocarbonyltriazolinone der allgemeinen Formel (I), wenn man
a) Triazolinone der allgemeinen Formel (II) 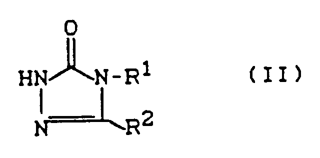 in welcher
   - R¹ und R²: die oben angegebenen Bedeutungen haben,
   mit Sulfonylisocyanaten der allgemeinen Formel (III)

   R³-SO₂-N=C=O (III)

   in welcher
   - R³: die oben angegebene Bedeutung hat,
   gegebenenfalls in Gegenwart eines Verdünnungsmittels umsetzt, oder wenn man
b) Triazolinon-Derivate der allgemeinen Formel (IV) 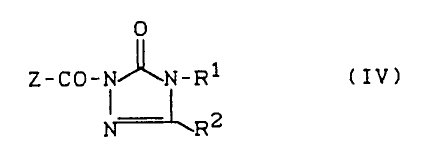 in welcher
   - R¹ und R²: die oben angegebenen Bedeutungen haben und
   - Z: für Chlor, C₁-C₄-Alkoxy, Benzyloxy oder Phenoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt, oder wenn man
c) Triazolinone der allgemeinen Formel (II) 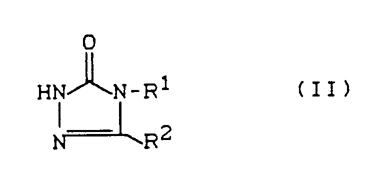 in welcher
   - R¹ und R²: die oben angegebenen Bedeutungen haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Chlor, C₁-C₄-Alkoxy, Benzyloxy oder Phenoxy steht,
   gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

Die neuen Sulfonylaminocarbonyltriazolinone der allgemeinen Formel (I) und ihre Salze zeichnen sich durch starke herbizide Wirksamkeit aus.

Überraschenderweise zeigen die neuen Verbindungen der Formel (I) erheblich bessere herbizide Wirkung als das strukturell ähnliche bekannte Herbizid 1-Isobutylaminocarbonyl-2-imidazolidinon (Isocarbamid).

Gegenstand der Erfindung sind vorzugsweise Verbindungen der Formel (I), in welcher
- R¹: für C₁-C₃-Alkoxy steht,
- R²: für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
- R³: für die Gruppierung 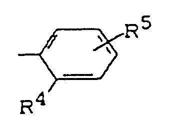 steht, worin
- R⁴: für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
- R⁵: für Wasserstoff, Fluor, Chlor oder Brom steht;
weiterhin
- R³: für den Rest 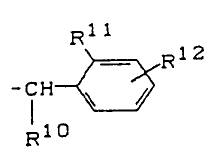 steht, worin
- R¹⁰: für Wasserstoff steht,
- R¹¹: für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
- R¹²: für Wasserstoff steht;
weiterhin
- R³: für den Rest 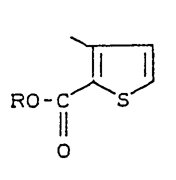 steht,
worin R für C₁-C₄-Alkyl steht, oder
für den Rest 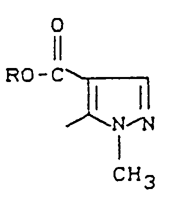 steht,
worin R für C₁-C₄-Alkyl steht,
sowie Salze dieser Verbindungen.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzyl-ammonium-Salze von Verbindungen der Formel (I), in welcher R¹, R² und R³ die oben vorzugsweise angegebenen Bedeutungen haben.

Gegenstand der Erfindung sind insbesondere Verbindungen der Formel (I), in welcher
- R¹: für C₁-C₃-Alkoxy steht,
- R²: für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
- R³: für die Gruppierung 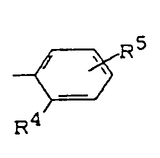 steht, worin
- R⁴: für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
- R⁵: für Wasserstoff, Fluor, Chlor oder Brom steht; oder
weiterhin
- R³: für den Rest 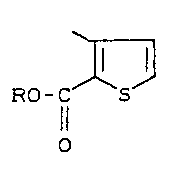 steht,
worin R für C₁-C₄-Alkyl steht,
sowie Salze dieser Verbindungen.

Beispiele für die erfindungsgemäßen Verbindungen sind in der nachstehenden Tabelle 1 aufgeführt - vgl. auch die Herstellungsbeispiele.

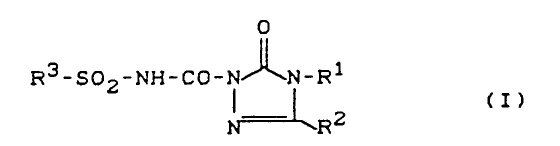

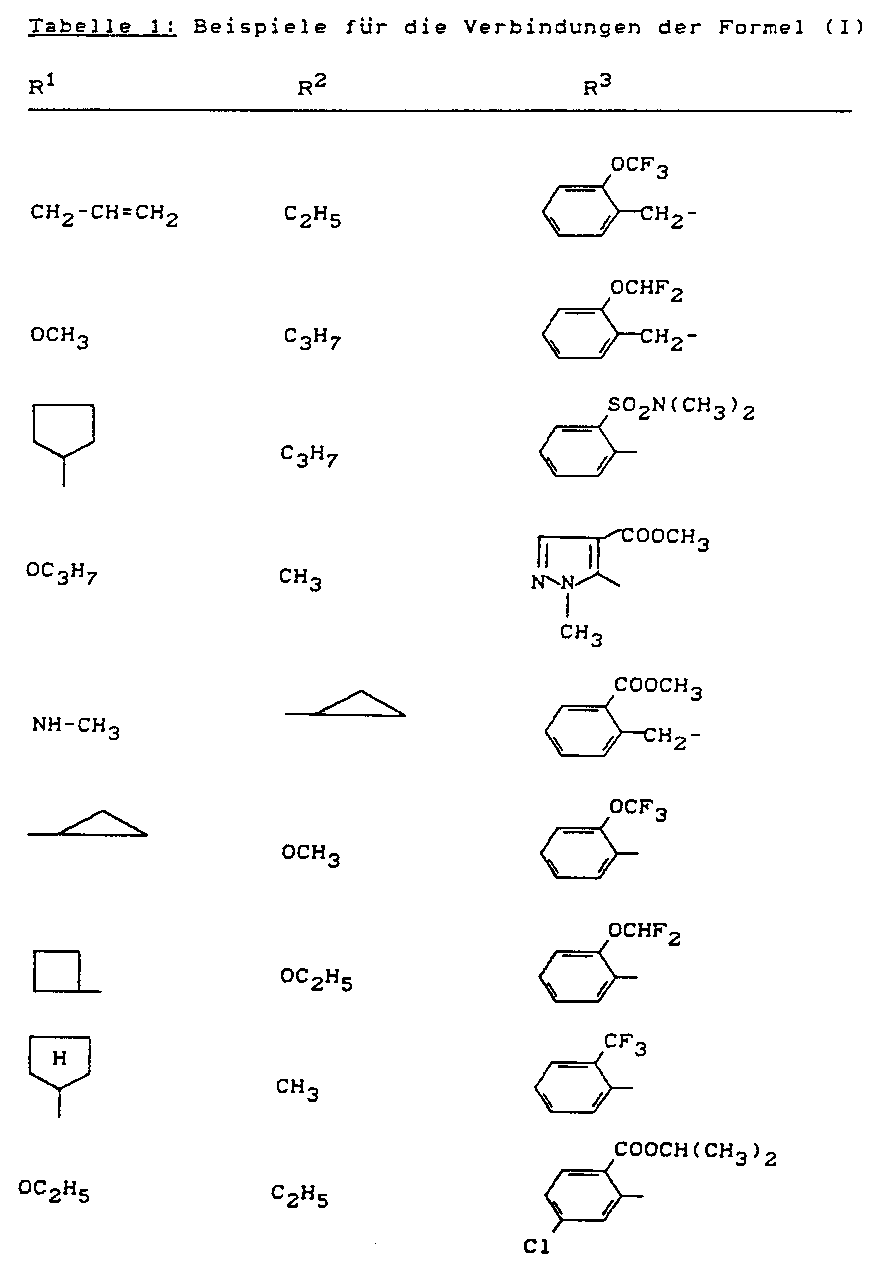

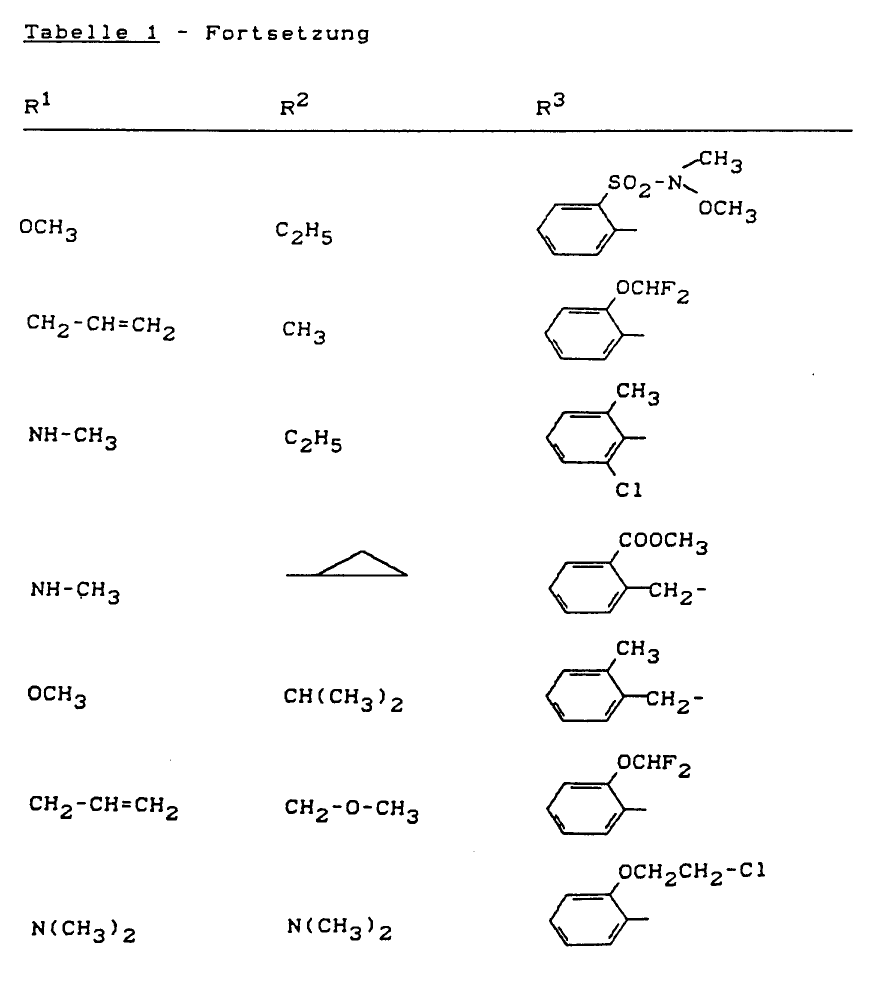

Verwendet man beispielsweise 2,6-Difluor-phenylisocyanat und 5-Ethyl-4-methoxy-2,4-dihydro-3H-1,2,4-triazol-3-on als Ausgangsstoffe, so kann der Reaktionsablauf beim erfindungsgemäßen Verfahren (a) durch folgendes Formelschema skizziert werden: 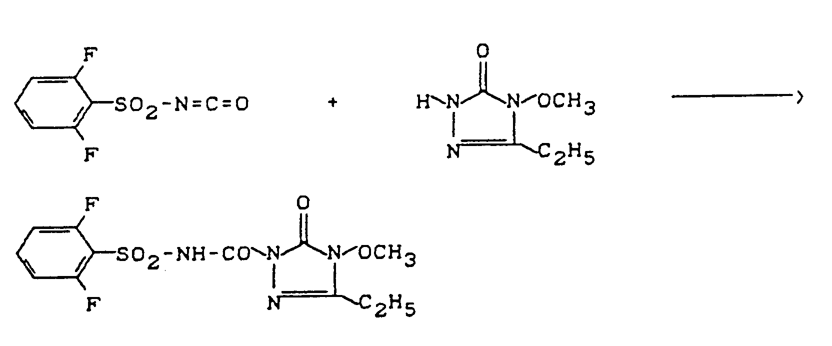
Verwendet man beispielsweise 2,6-Difluor-benzolsulfonsäureamid und 2-Chlorcarbonyl-5-ethyl-4-methoxy-2,4-dihydro-3H-1,2,4-triazol-3-on als Ausgangsstoffe, so kann der Reaktionsablauf beim erfindungsgemäßen Verfahren (b) durch folgendes Formelschema skizziert werden:

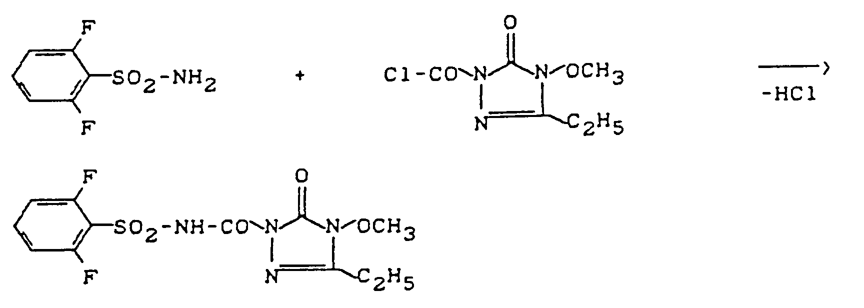

Verwendet man beispielsweise N-Methoxycarbonyl-2,6-difluor-benzolsulfonsäureamid und 5-Ethyl-4-methoxy-2,4-dihydro-3H-1,2,4-triazol-3-on als Ausgangsstoffe, so kann der Reaktionsablauf beim erfindungsgemäßen Verfahren (c) durch das folgende Formelschema skizziert werden: 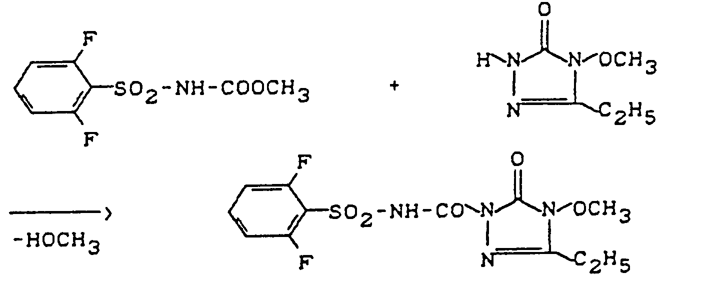
Die bei den erfindungsgemäßen Verfahren (a) und (c) zur Herstellung von Verbindungen der Formel (I) als Ausgangsstoffe zu verwendenden Triazolinone sind durch die Formel (II) allgemein definiert.

In Formel (II) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Beispiele für die Ausgangsstoffe der Formel (II) sind in der nachstehenden Tabelle 2 aufgeführt.

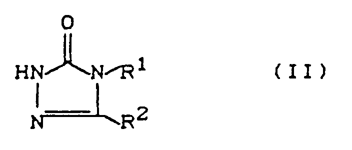

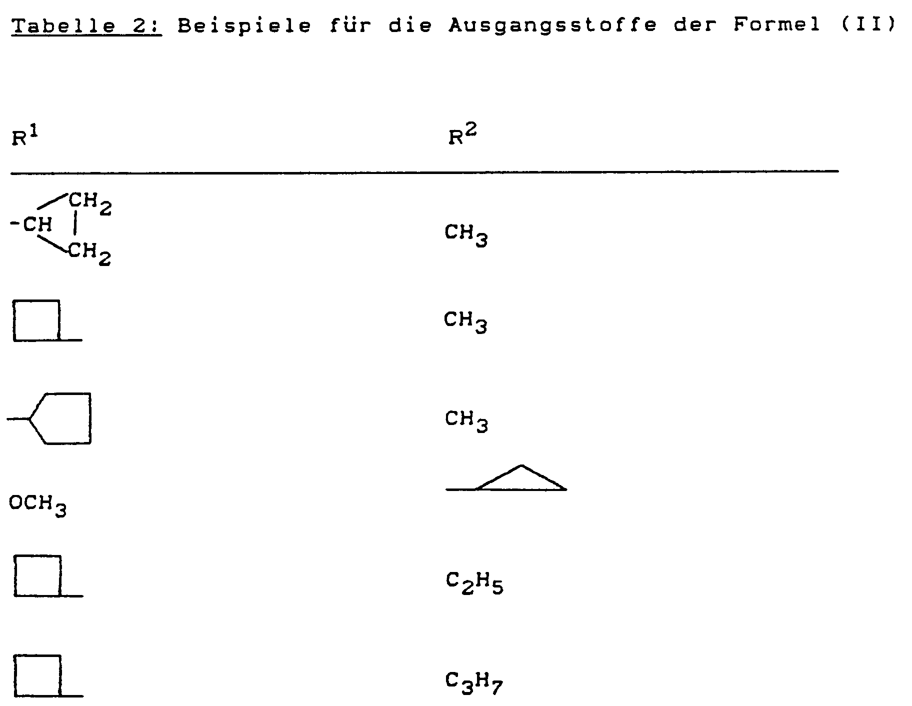

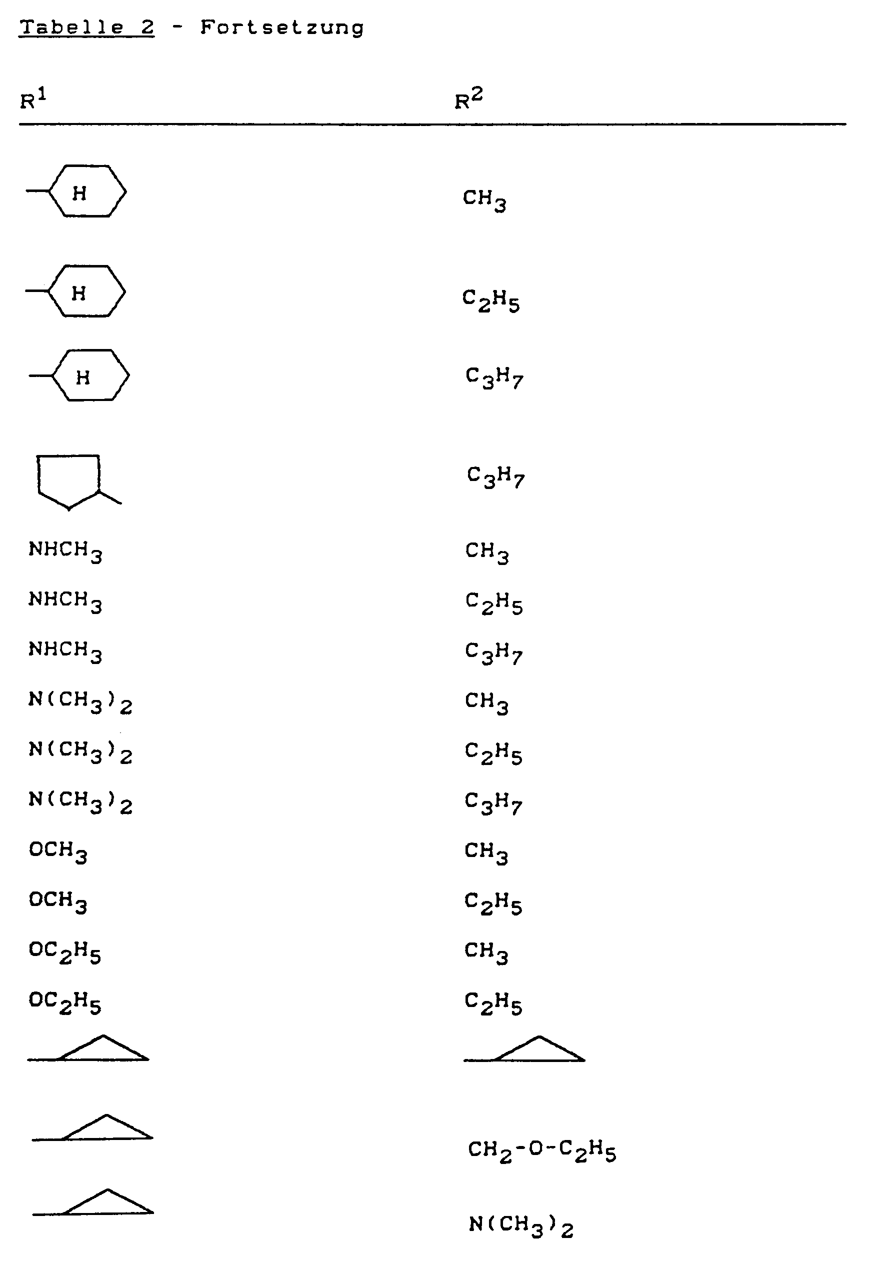

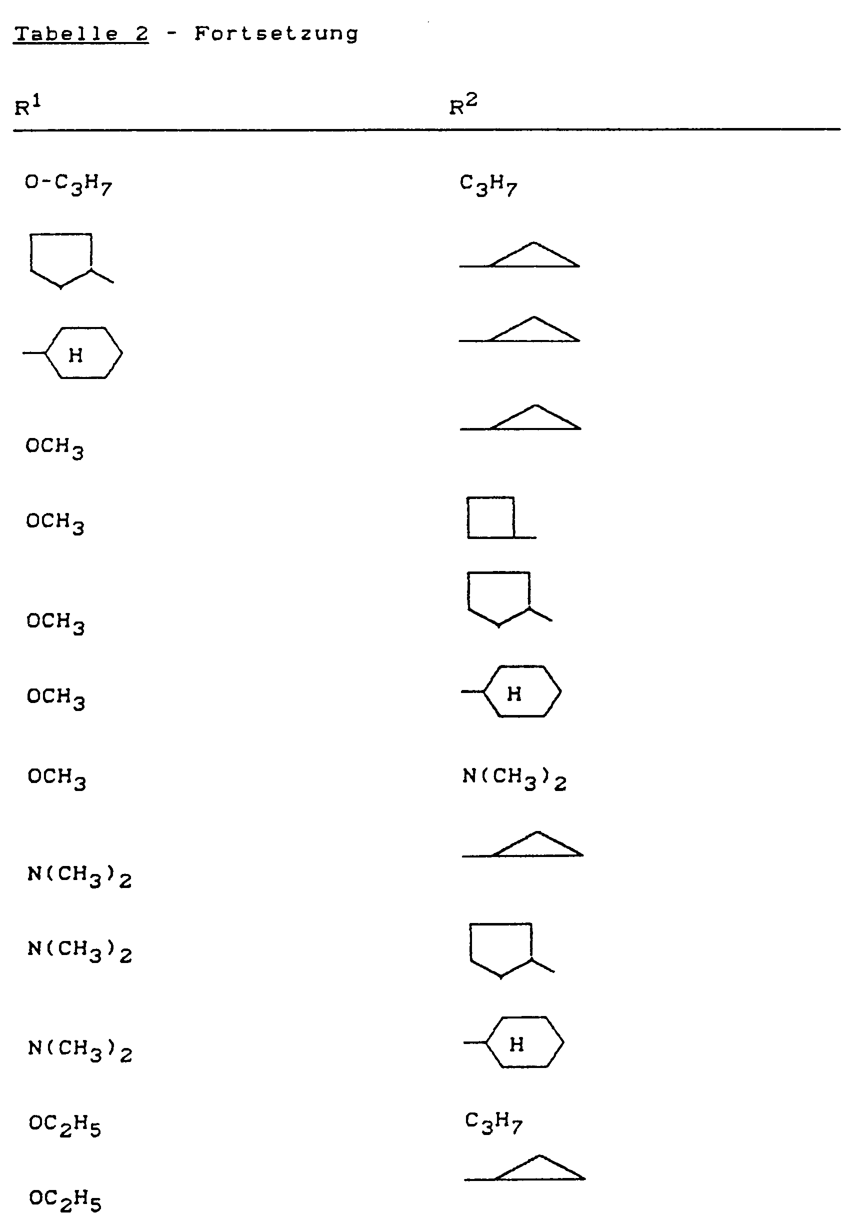

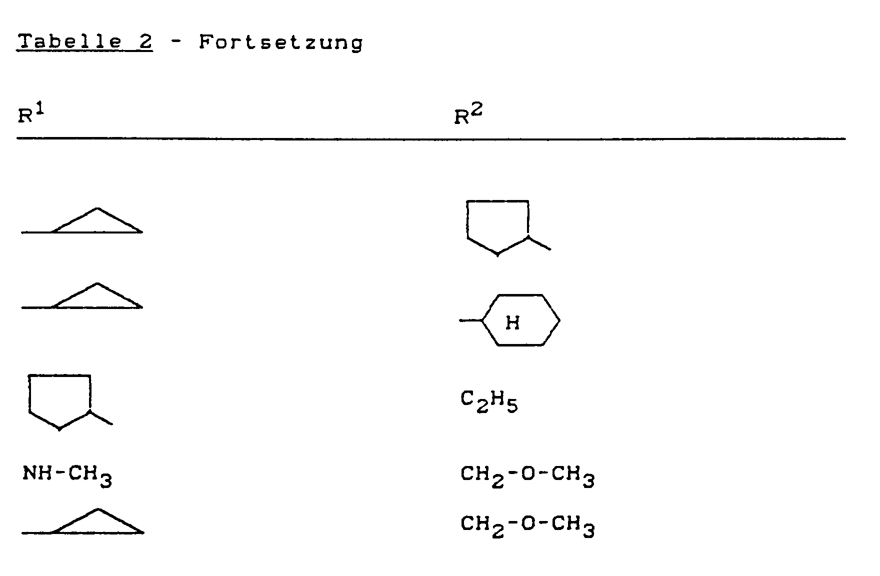

Die Ausgangsstoffe der Formel (II) sind bekannt und/oder können nach an sich bekannten Verfahren hergestellt werden (vgl. Chem. Ber. 90 (1957), 909-921; ibid. 98 (1965), 3025-3099; J. Heterocycl. Chem. 15 (1978), 237-240; Tetrahedron 32 (1976), 2347-2352; Helv. Chim. Acta 63 (1980), 841-859; J. Chem. Soc. C 1967, 746-751; EP-A 283876; EP-A 294666; EP-A 301946; EP-A 298371; DE-P 3839206/LeA 26538 vom 19.11.1988; DE-P 3916207/LeA 26849 vom 18.05.1989; DE-P 3916208/LeA 26850 vom 18.05.1989; J. Chem. Soc. C 1970, 26-34; DE-P 3916930/LeA 26886 vom 24.05.1989).

Man erhält die Triazolinone der Formel (II) beispielsweise, wenn man
α) Oxadiazolinone der allgemeinen Formel (VII) 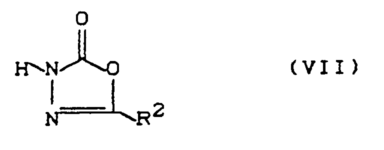 in welcher
   - R²: die oben angegebene Bedeutung hat,
   mit Aminoverbindungen der allgemeinen Formel (VIII)

   H₂N-R¹ (VIII)

   in welcher
   - R¹: die oben angegebene Bedeutung hat,
   gegebenenfalls in Gegenwart eines Verdünnungsmittels, wie z.B. Wasser, bei Temperaturen zwischen 20°C und 120°C umsetzt und die hierbei gebildeten Hydrazinderivate der allgemeinen Formel (IX) 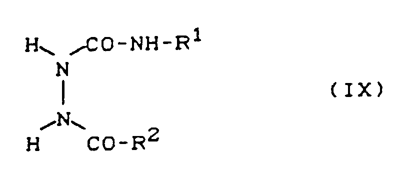 in welcher
   - R¹ und R²: die oben angegebenen Bedeutungen haben,
   nach üblichen Methoden isoliert (vgl. die Herstellungsbeispiele) und - oder gegebenenfalls auch ohne Zwischenisolierung - die Verbindungen der Formel (IX), gegebenenfalls in Gegenwart eines basischen Kondensationshilfsmittels, wie z.B. Natriumhydroxid, und gegebenenfalls in Gegenwart eines Verdünnungsmittels, wie z.B. Wasser, bei Temperaturen zwischen 20°C und 120°C zu den Verbindungen der Formel (II) kondensiert (vgl. EP-A 301946, DE-OS 3743493 und die Herstellungsbeispiele), oder wenn man
β) Aminoverbindungen der allgemeinen Formel (VIII)

   H₂N-R¹ (VIII)

   in welcher
   - R¹: die oben angegebene Bedeutung hat,
   mit Kohlensäurederivaten, wie z.B. Diphenylcarbonat, anschließend mit Hydrazin oder Hydrazinhydrat und schließlich mit einem Carbonsäure- bzw. Kohlensäure-Derivat der allgemeinen Formel (X)

   (RO)₃C-R² (X)

   in welcher
   - R²: die oben angegebene Bedeutung hat und
   - R: für Niederalkyl steht,
   gegebenenfalls in Gegenwart eines Verdünnungsmittels, wie z.B. Ethylenchlorid, bei Temperaturen zwischen 0°C und 150°C umsetzt (vgl. DE-P 3 920 270 vom 21.06.1989, DE-P 3 928 662 vom 30.08.1989 und die Herstellungsbeispiele).

Neu sind die Triazolinone der allgemeinen Formel (IIa) 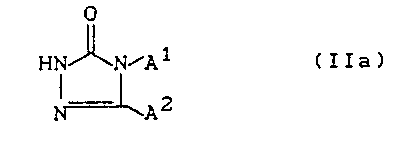
in welcher
- A¹: für Allyl, für Cyclopropyl, für C₁-C₃-Alkoxy oder für Di-(C₁-C₂-alkyl)-amino steht und
- A²: für gegebenenfalls durch Fluor und/oder Chlor, Methoxy oder Ethoxy substituiertes C₁-C₄-Alkyl oder für Cyclopropyl steht,
ausgenommen jedoch die Verbindungen
4-Cyclopropyl-5-methyl-2,4-dihydro-3H-1,2,4-triazol-3-on und 4-Cyclopropyl-5-ethyl-2,4-dihydro-3H-1,2,4-triazol-3-on.

Man erhält die neuen Triazolinone der Formel (IIa) beispielsweise, wenn man entweder Oxadiazolinone der allgemeinen Formel (VIIa) 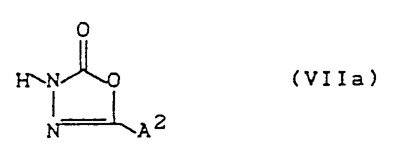
in welcher
- A²: die oben angegebene Bedeutung hat,
mit Aminoverbindungen der allgemeinen Formel (VIIIa)

H₂N-A¹ (VIIIa)

in welcher
- A¹: die oben angegebene Bedeutung hat,
analog zum oben unter (α) beschriebenen Verfahren umsetzt oder wenn man
Aminoverbindungen der allgemeinen Formel (VIIIa)

H₂N-A¹ (VIIIa)

mit Kohlensäurederivaten, anschließend mit Hydrazin oder Hydrazinhydrat und schließlich mit einem Carbonsäure- bzw. Kohlensäure-Derivat der allgemeinen Formel (Xa)

(RO)₃C-A² (Xa)

in welcher
- A² und R: die oben angegebenen Bedeutungen haben,
analog zum oben unter (β) beschriebenen Verfahren umsetzt (vgl. ferner auch die Herstellungsbeispiele).

Die zur Herstellung der Triazolinone der Formeln (II) bzw. (IIa) als Ausgangsstoffe zu verwendenden Verbindungen der Formeln (VII), (VIIa), (VIII), (VIIIa) und (X) bzw. (Xa) sind bekannt (vgl. Helv. Chim. Acta 55 (1972), 1174; EP-A 301946; DE-OS 3743493).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In Formel (III) hat R³ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R³ angegeben wurde.

Als Beispiele für die Ausgangsstoffe der Formel (III) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-phenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonylisocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 80 °C.

Das erfindungsgemäße Verfahren (a) wird im allgemeinen bei Normaldruck durchgeführt.

Zur Durchführung des erfindungsgemäßen Verfahrens (a) setzt man je Mol Triazolinon der Formel (II) im allgemeinen zwischen 1 und 3 Mol, vorzugsweise zwischen 1 und 2 Mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt, eingeengt und das im Rückstand verbleibende Rohprodukt mit einem geeigneten Lösungsmittel, wie z. B. Diethylether, zur Kristallisation gebracht. Das kristallin angefallene Produkt der Formel (I) wird durch Absaugen isoliert.

Die beim erfindungsgemäßen Verfahren (b) zur Herstellung von Verbindungen der Formel (I) als Ausgangsstoffe zu verwendenden Triazolinon-Derivate sind durch die Formel (IV) allgemein definiert.

In Formel (IV) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden und
- Z: steht für Chlor, C₁-C₄-Alkoxy, Benzyloxy oder Phenoxy, vorzugsweise für Methoxy oder Phenoxy.

Mögliche Beispiele für die Ausgangsstoffe der Formel (IV) sind die aus den in Tabelle 2 aufgeführten Verbindungen der Formel (II) und Phosgen, Chlorameisensäure-methylester, Chlorameisensäure-benzylester, Chlorameisensäurephenylester oder Diphenylcarbonat herzustellenden Verbindungen der Formel (IV).

Die Ausgangsstoffe der Formel (IV) sind bekannt und/oder können nach an sich bekannten Verfahren hergestellt werden (vgl. EP-A 283876; EP-A 294666; EP-A 298371).

Man erhält die Triazolinon-Derivate der Formel (IV) beispielsweise, wenn man Triazolinone der allgemeinen Formel (II) 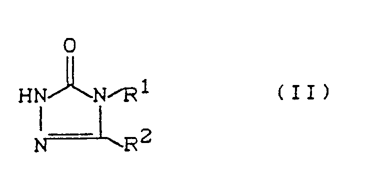
in welcher
- R¹ und R²: die oben angegebenen Bedeutungen haben,
mit Kohlensäurederivaten der allgemeinen Formel (XI)

Z-CO-Z¹ (XI)

in welcher
- Z: die oben angegebene Bedeutung hat und
- Z¹: für eine Abgangsgruppe wie Chlor, Methoxy, Benzyloxy oder Phenoxy steht,
gegebenenfalls in Gegenwart eines Verdünnungsmittels, wie z.B. Tetrahydrofuran, und gegebenenfalls in Gegenwart eines Säureakzeptors, wie z.B. Natriumhydrid oder Kalium-tert-butylat, bei Temperaturen zwischen -20°C und +100°C umsetzt (vgl. die Herstellungsbeispiele).

Die beim erfindungsgemäßen Verfahren (b) zur Herstellung von Verbindungen der Formel (I) weiter als Ausgangsstoffe zu verwendenden Sulfonsäureamide sind durch die Formel (V) allgemein definiert.

In Formel (V) hat R³ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R³ angegeben wurde.

Als Beispiele für die Ausgangsstoffe der Formel (V) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-benzolsulfonsäureamid, 2-Fluor-, 2-Chlor-, 2-Difluormethoxy-, 2-Trifluormethoxy-, 2-Methoxycarbonyl- und 2-Ethoxycarbonyl-phenylmethansulfonsäureamid, 2-Methoxycarbonyl-3-thiophensulfonsäureamid, 4-Methoxycarbonyl-und 4-Ethoxycarbonyl-1-methyl-pyrazol-5-sulfonsäureamid.

Die Sulfonsäureamide der Formel (V) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Betracht, wie sie beispielsweise oben für das erfindungsgemäße Verfahren (a) angegeben sind.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden Triazolinone der Formel (II) sind bereits als Ausgangsstoffe für das erfindungsgemäße Verfahren (a) beschrieben worden.

Die beim erfindungsgemäßen Verfahren (c) zur Herstellung von Verbindungen der Formel (I) weiter als Ausgangsstoffe zu verwendenden Sulfonsäureamid-Derivate sind durch die Formel (VI) allgemein definiert.

In Formel (VI) haben R³ und Z vorzugsweise bzw. insbesondere diejenigen Bedeutungen, die bereits oben im Zusammenhang mit der Beschreibung der erfindungsgemäßen Verbindungen der Formel (I) bzw. (IV) vorzugsweise bzw. als insbesondere bevorzugt für R³ und Z angegeben wurden.

Das erfindungsgemäße Verfahren (c) wird vorzugsweise unter Verwendung von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (c) wird gegebenenfalls in Gegenwart eines Säureakzeptors durchgeführt. Es kommen hierbei die gleichen Säurebindemittel in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Zur Überführung in Salze werden die Verbindungen der Formel (I) mit geeigneten Salzbildnern, wie z.B. Natrium- oder Kalium-hydroxid, -methylat oder -ethylat, Ammoniak, Isopropylamin, Dibutylamin oder Triethylamin, in geeigneten Verdünnungsmitteln, wie z.B. Wasser, Methanol oder Ethanol, verrührt. Die Salze können - dann gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern sowohl im Vorauflauf- als auch im Nachauflauf-Verfahren. Sie sind deutlich wirksamer als z.B. Isocarbamid.

In gewissem Umfang zeigen die erfindungsgemäßen Verbindungen auch fungizide Wirkung, z.B. gegen echte Mehltaupilze und gegen Apfelschorf sowie gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2,6-Dichlorbenzonitril (DICHLOBENIL); 2-[4-(2,4-Dichlorphenoxy)-phenoxy]-propionsäure, deren Methyl-oder deren Ethylester (DICLOFOP); 2-[(2-Chlorphenyl)-methyl]-4,4-dimethylisoxazolidin-3-on (DIMETHAZONE); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); 5-(2-Chlor-4-trifluormethyl-phenoxy)-N-methylsulfonyl-2-nitrobenzamid (FOMESAFEN); N-Phosphonomethyl-glycin (GLYPHOSATE); Methyl-2-[4,5-dihydro-4-methyl-4-(1-methylethyl)-5-oxo-1H-imidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl)-benzoesäure oder deren Methylester (METSULFURON); 1-(3-Trifluormethyl-phenyl)-4-methylamino-5-chlor-6-pyridazon (NORFLURAZON); N-(1-Ethylpropyl)-3,4-dimethyl-2,6-dinitroanilin (PENDIMETHALIN); 0-(6-Chlor-3-phenyl-pyridazin-4-yl)-S-octylthiocarbamat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); Methyl-2-{[(4,6-dimethyl-2-pyrimidinyl)-aminocarbonyl]-aminosulfonyl}-benzoat (SULFOMETURON); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

### Herstellungsbeispiele:

### Beispiel 1

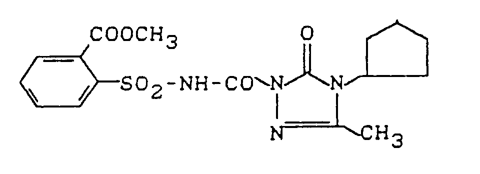

### (Verfahren (a))

3,0 g (17,95 mMol) 4-Cyclopentyl-5-methyl-2,4-dihydro-3H-1,2,4-triazol-3-on werden in 60 ml Acetonitril gelöst und unter Rühren werden 6,9 g (28,6 mMol) 2-Methoxycarbonyl-phenylsulfonylisocyanat, gelöst in 20 ml Acetonitril zu dieser Lösung gegeben. Das Reaktionsgemisch wird 6 Stunden bei 20°C gerührt und dann eingeengt. Der verbleibende Rückstand wird mit Diethylether verrührt und das kristallin angefallene Produkt durch Absaugen isoliert.

Man erhält 6,6 g (90% der Theorie) 4-Cyclopentyl-5-methyl-2-(2-methoxycarbonyl-phenylsulfonyl-aminocarbonyl)-2,4-dihydro-3H-1,2,4-triazol-3-on vom Schmelzpunkt 146°C.

Analog zu Beispiel 1 sowie entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

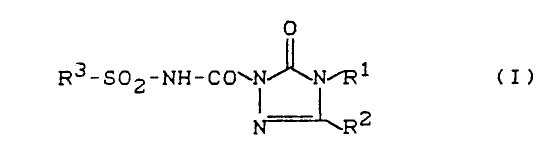

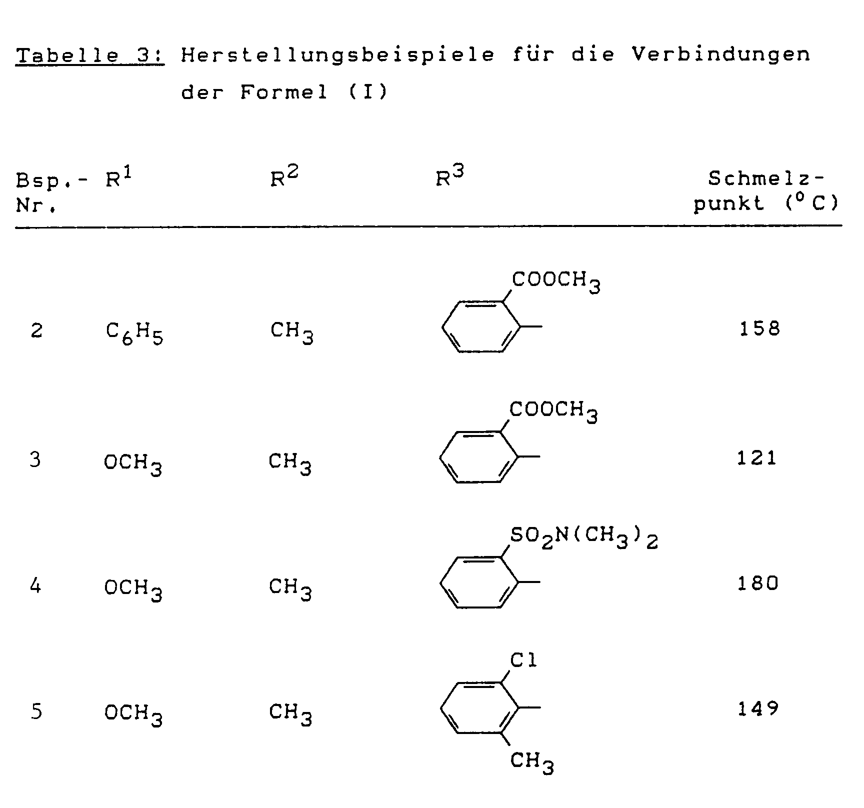

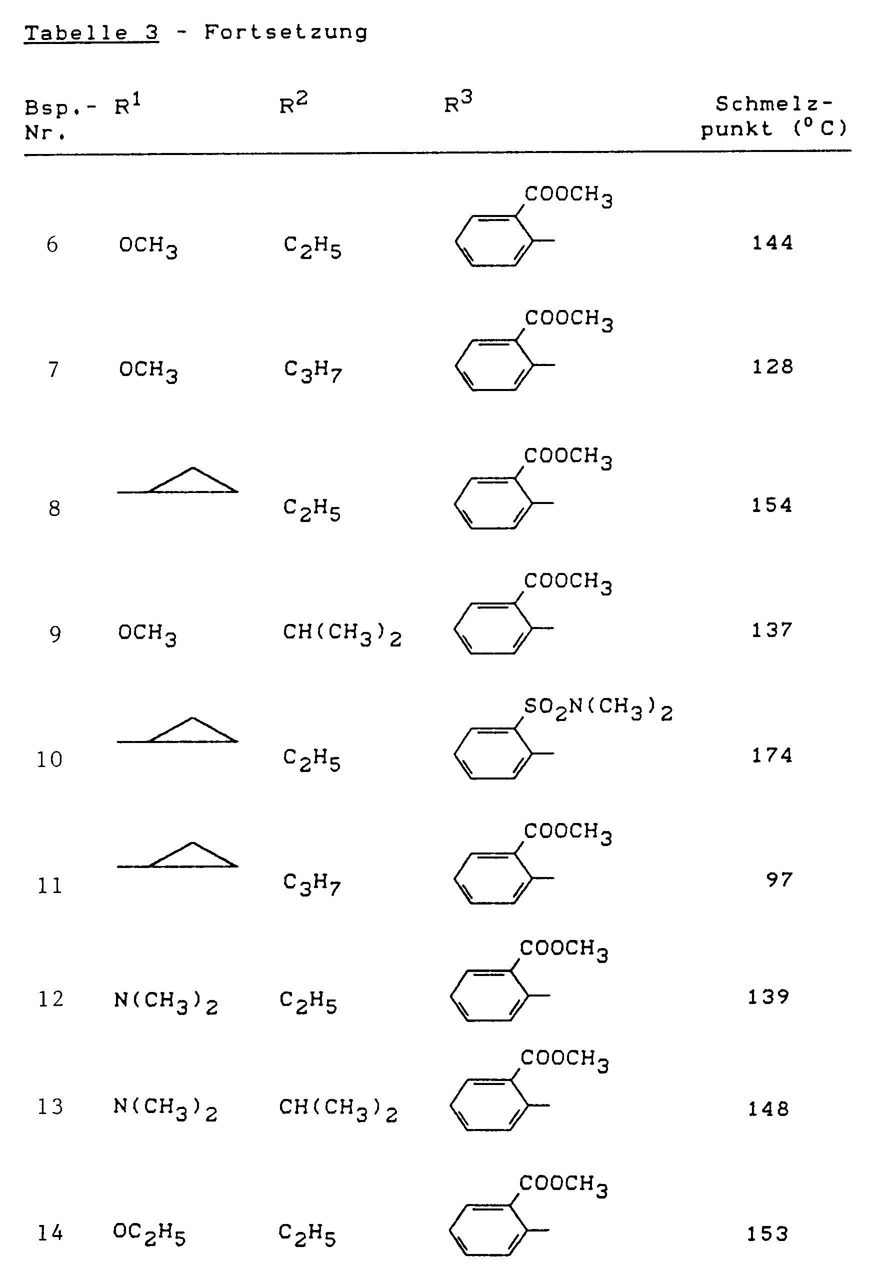

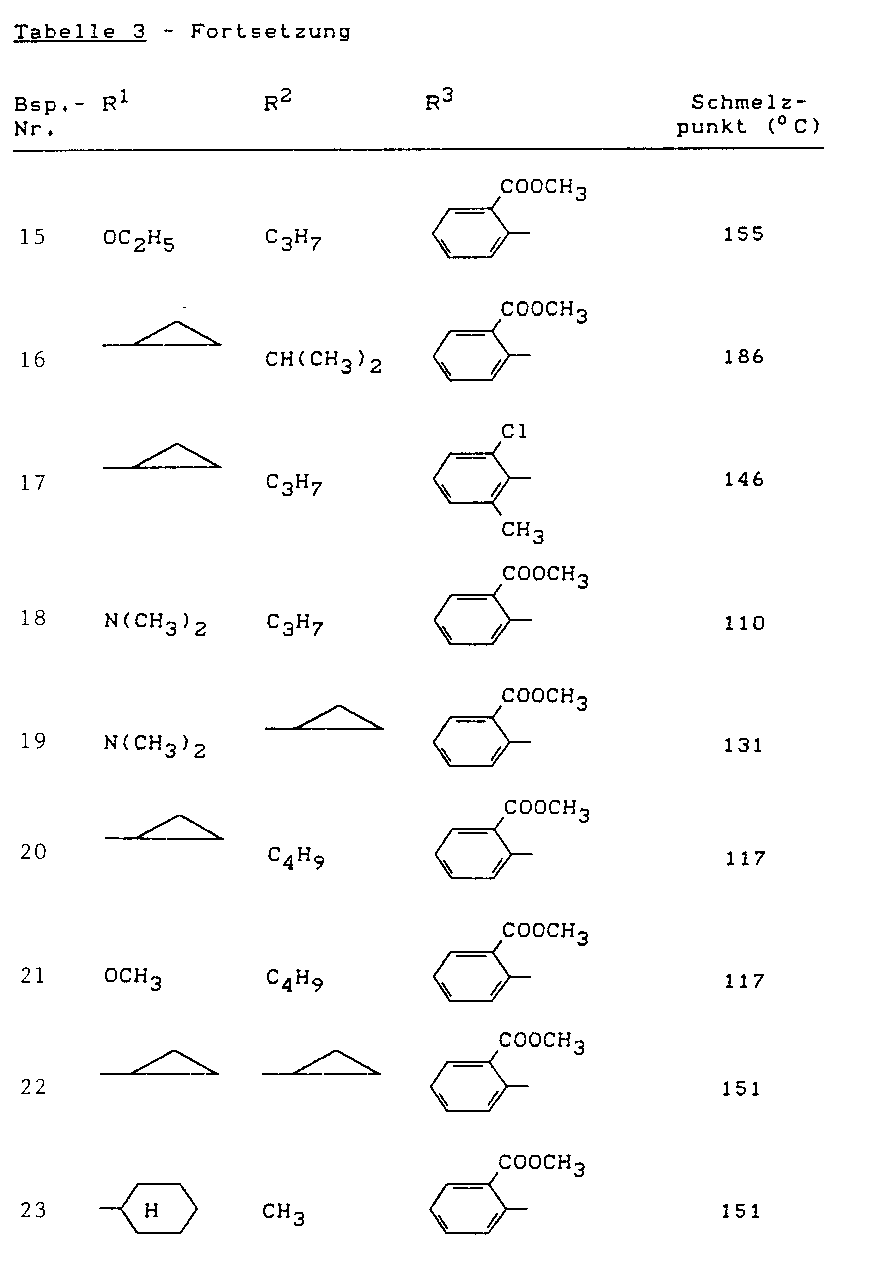

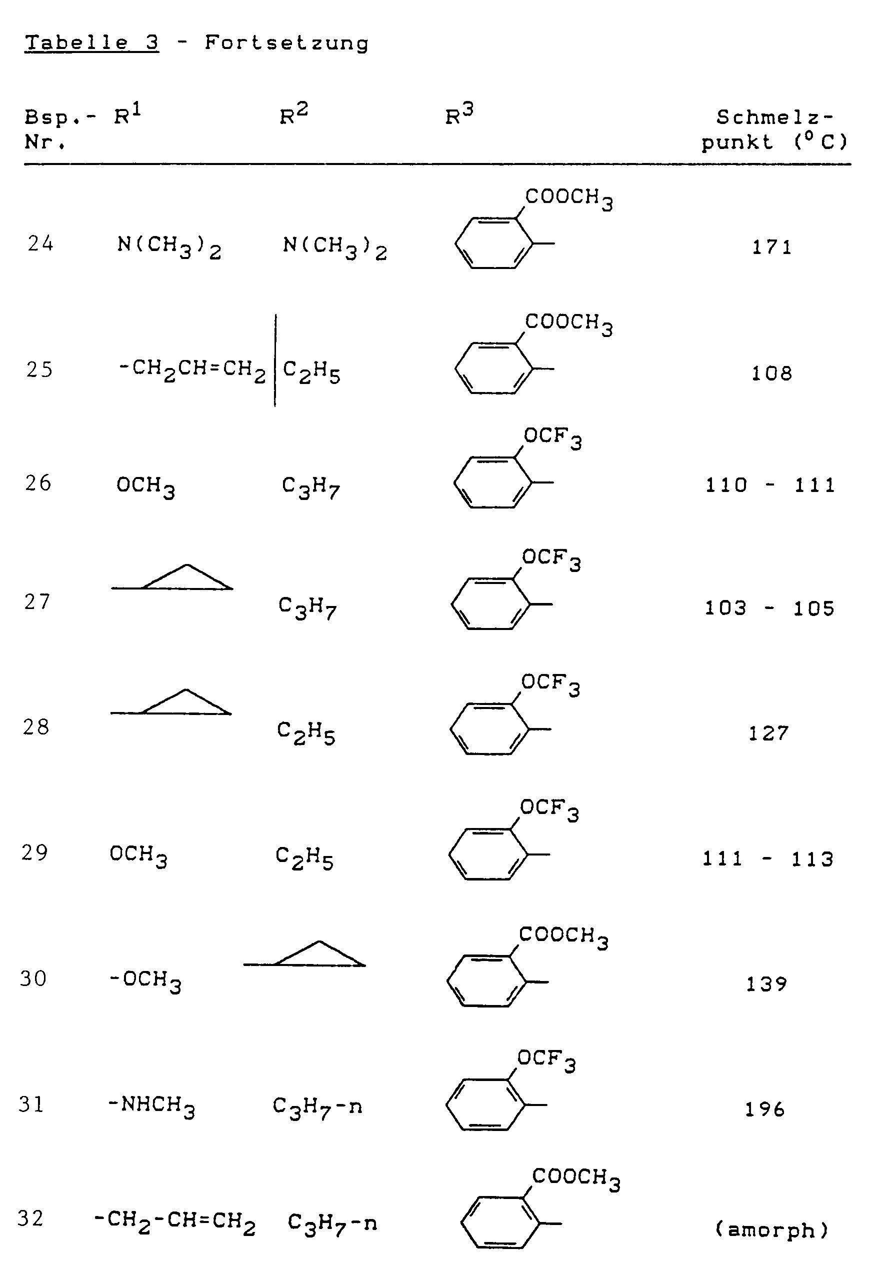

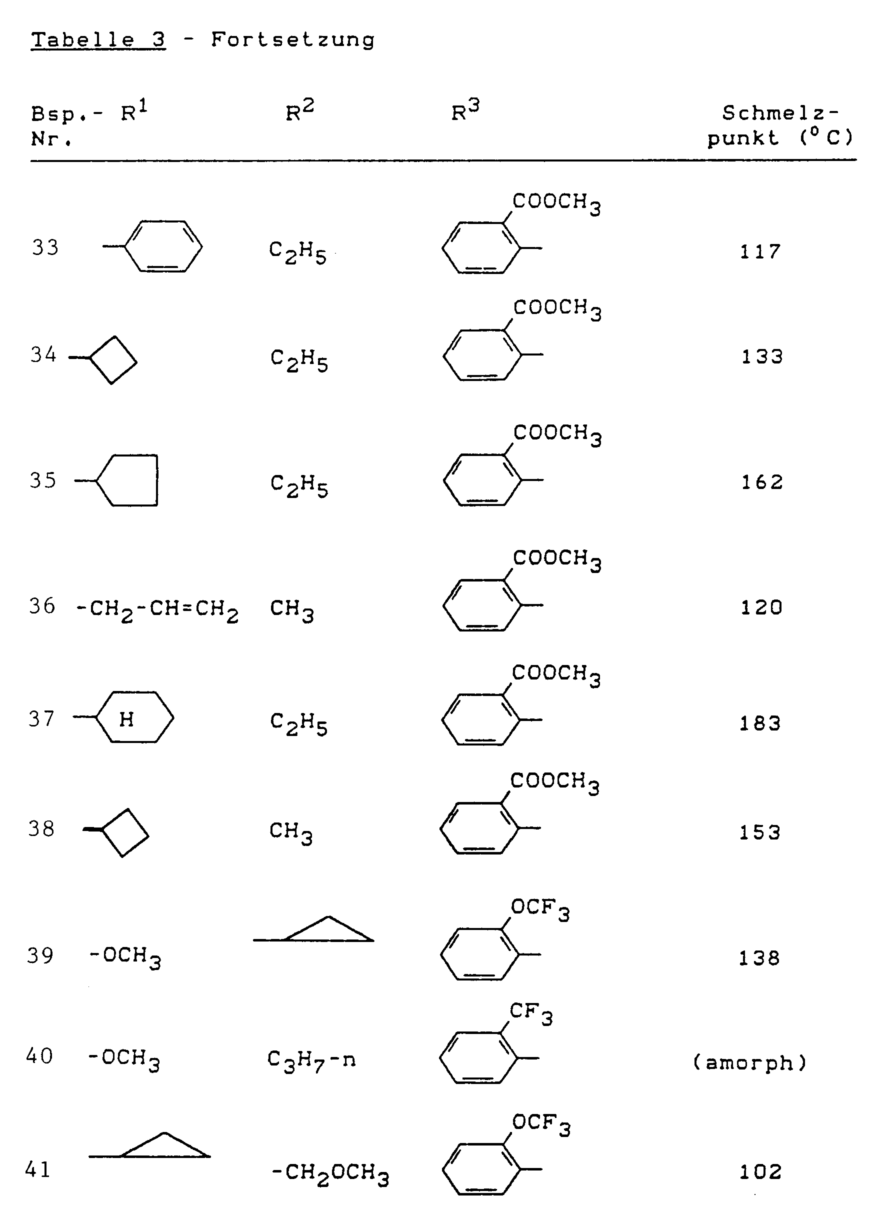

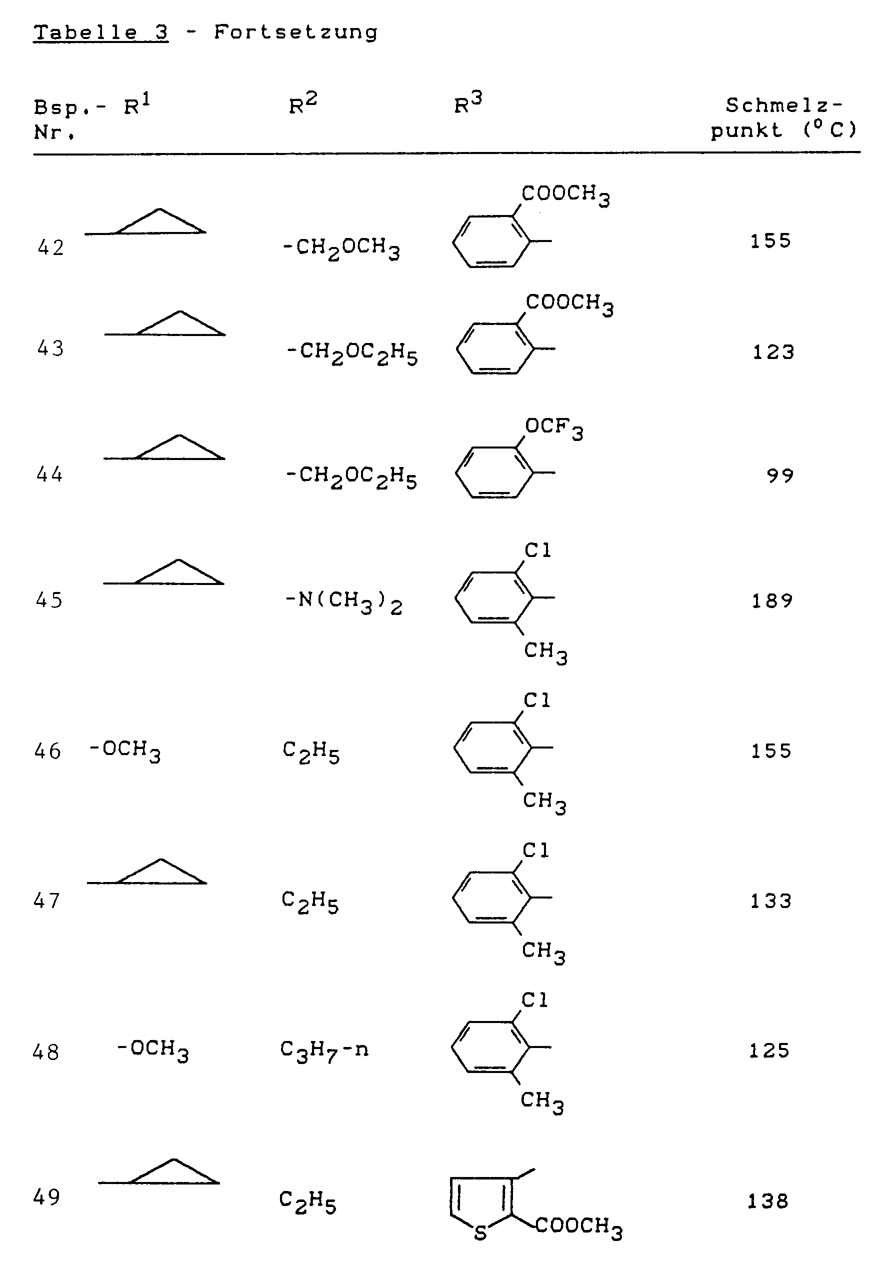

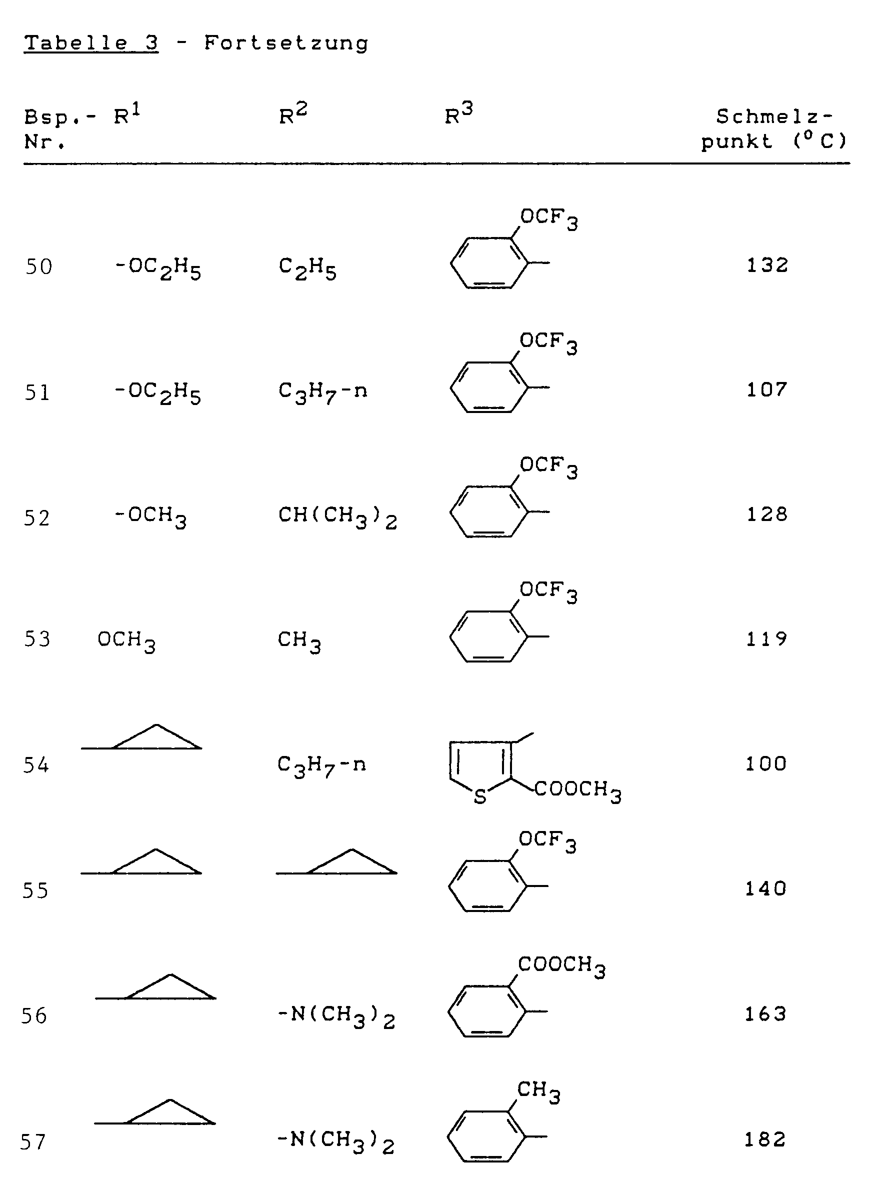

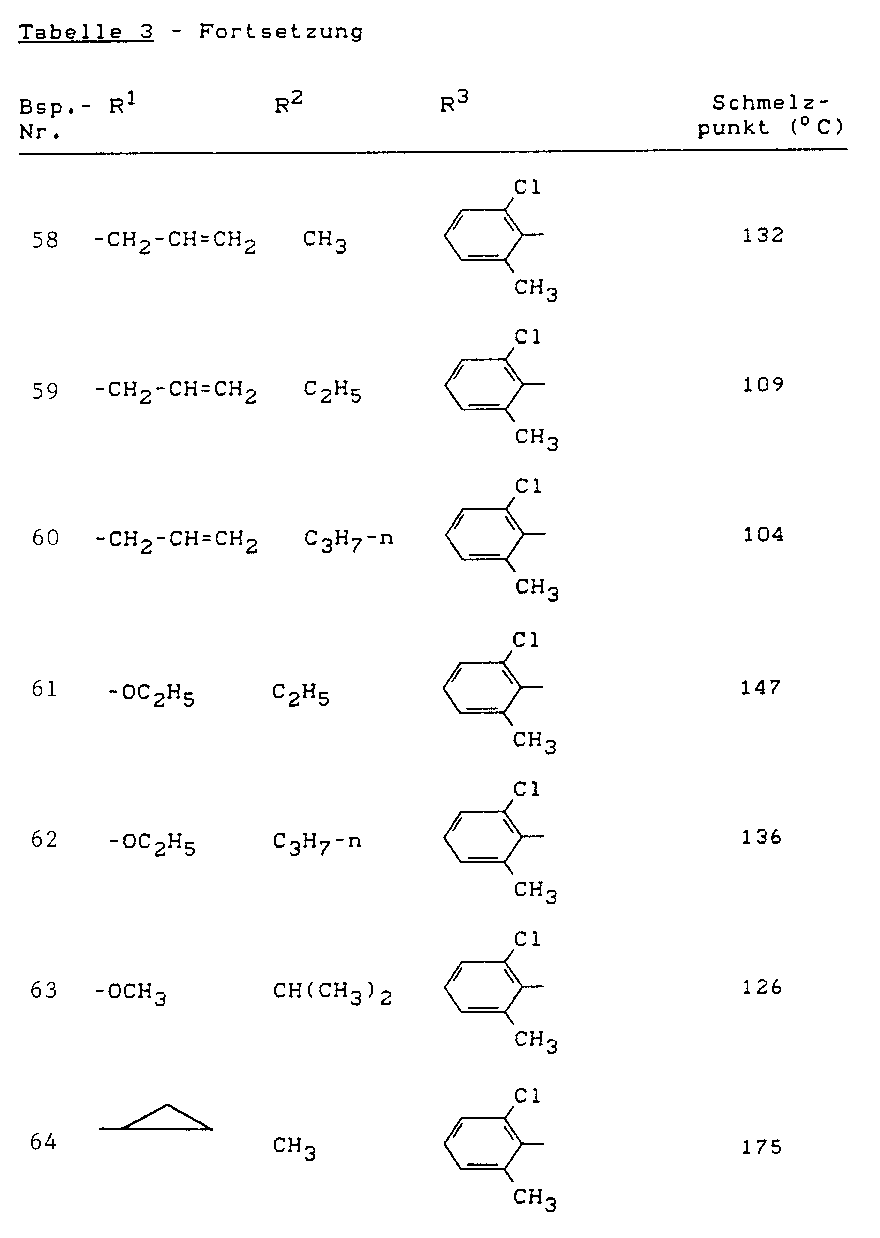

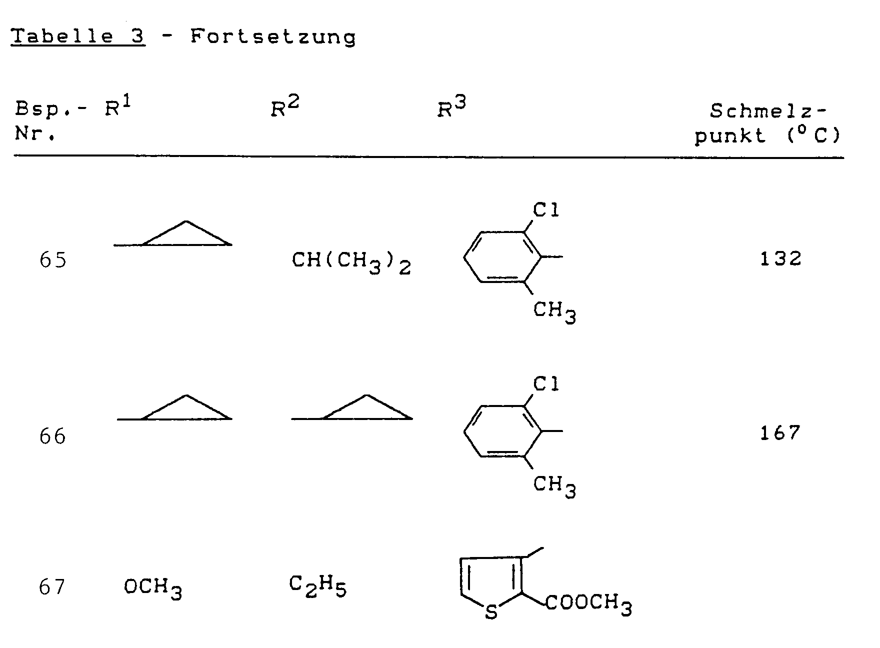

### Ausgangsstoffe der Formel (II):

### Beispiel (II-1)

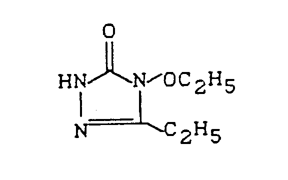

### Stufe 1:

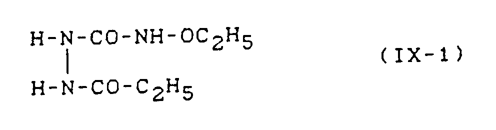

Eine Mischung aus 68,5 g (0,60 Mol) 5-Methyl-1,3,4-oxadiazolin-2-on, 45,8 g (0,75 Mol) O-Ethyl-hydroxylamin und 400 ml Wasser wird 12 Stunden unter Rückfluß erhitzt und anschließend eingeengt. Der Rückstand wird in Ethanol aufgenommen und erneut eingeengt. Der hierbei erhaltene Rückstand wird mit Diethylether verrührt und das kristallin angefallene Produkt durch Absaugen isoliert.

Man erhält 77,5 g (74% der Theorie) 1-Ethoxyaminocarbonyl-2-propionyl-hydrazin vom Schmelzpunkt 122°C.

### Stufe 2:

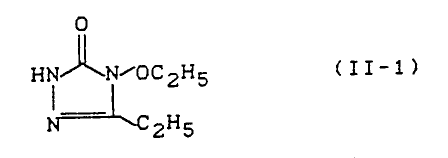

Eine Mischung aus 75,5 g (0,43 Mol) 1-Ethoxyaminocarbonyl-2-propionyl-hydrazin, 17,5 g (0,44 Mol) Natriumhydroxid und 300 ml Wasser wird 12 Stunden unter Rückfluß erhitzt. Nach dem Erkalten wird durch Zugabe von konzentrierter Salzsäure ein pH-Wert zwischen 3 und 4 eingestellt und eingeengt. Der Rückstand wird mit Essigsäureethylester verrührt und das ungelöst gebliebene Natriumchlorid durch Absaugen abgetrennt. Das Filtrat wird eingeengt, der Rückstand mit Diethylether verrührt und das kristallin angefallene Produkt durch Absaugen isoliert.

Man erhält 37 g (55% der Theorie) 4-Ethoxy-5-ethyl-2,4-dihydro-3H-1,2,4-triazol-3-on vom Schmelzpunkt 93°C.

Analog zu Beispiel (II-1) können beispielsweise auch die in der nachstehenden Tabelle 4 aufgeführten Verbindungen der Formeln (II) und (IIa) hergestellt werden.

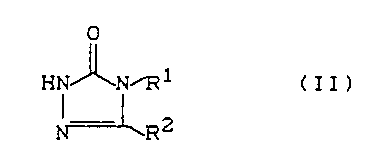

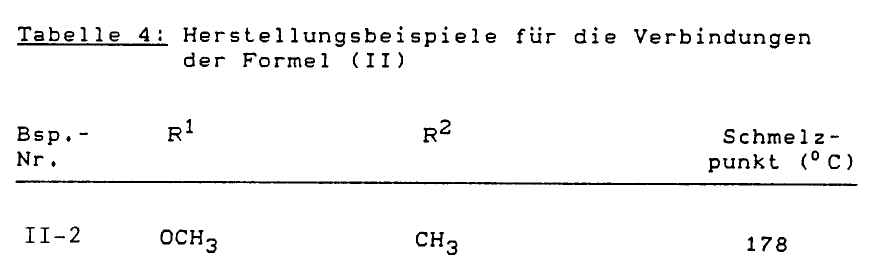

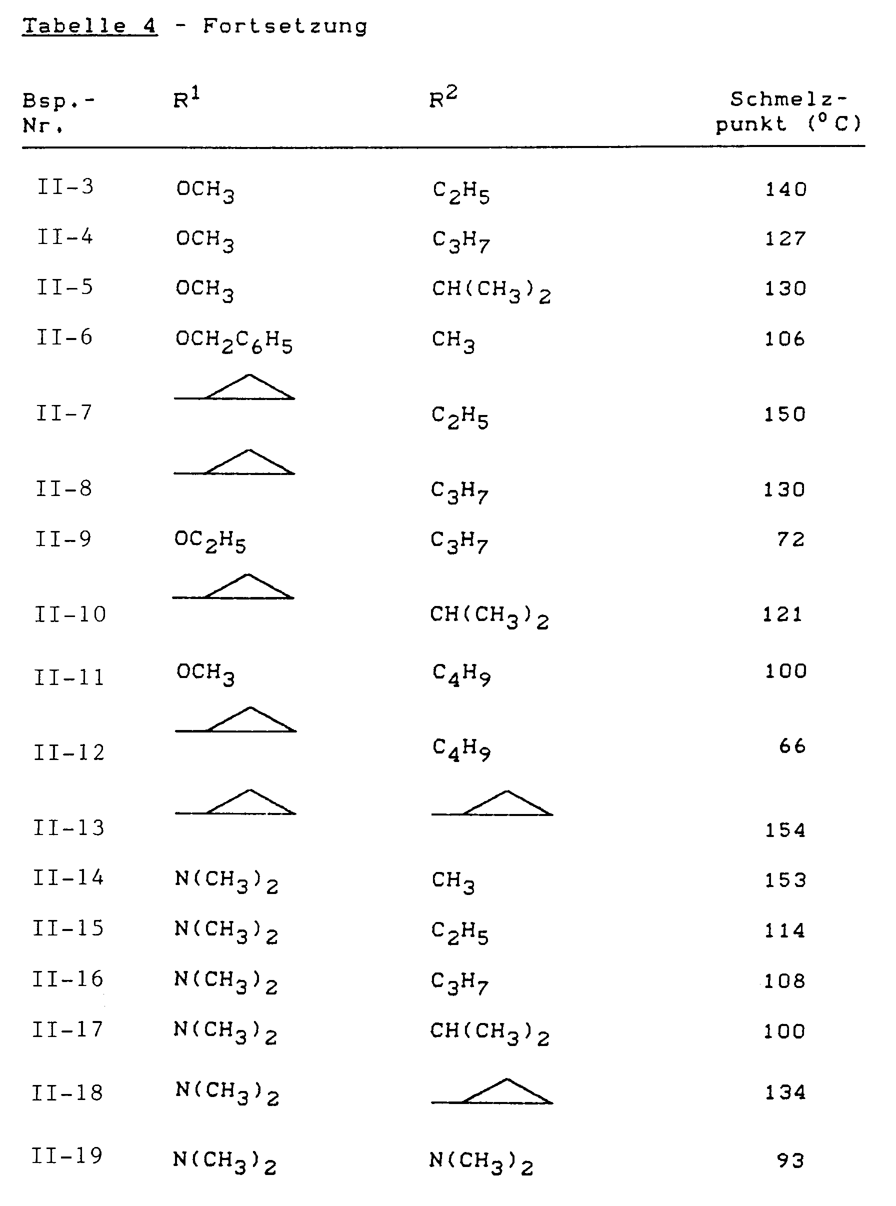

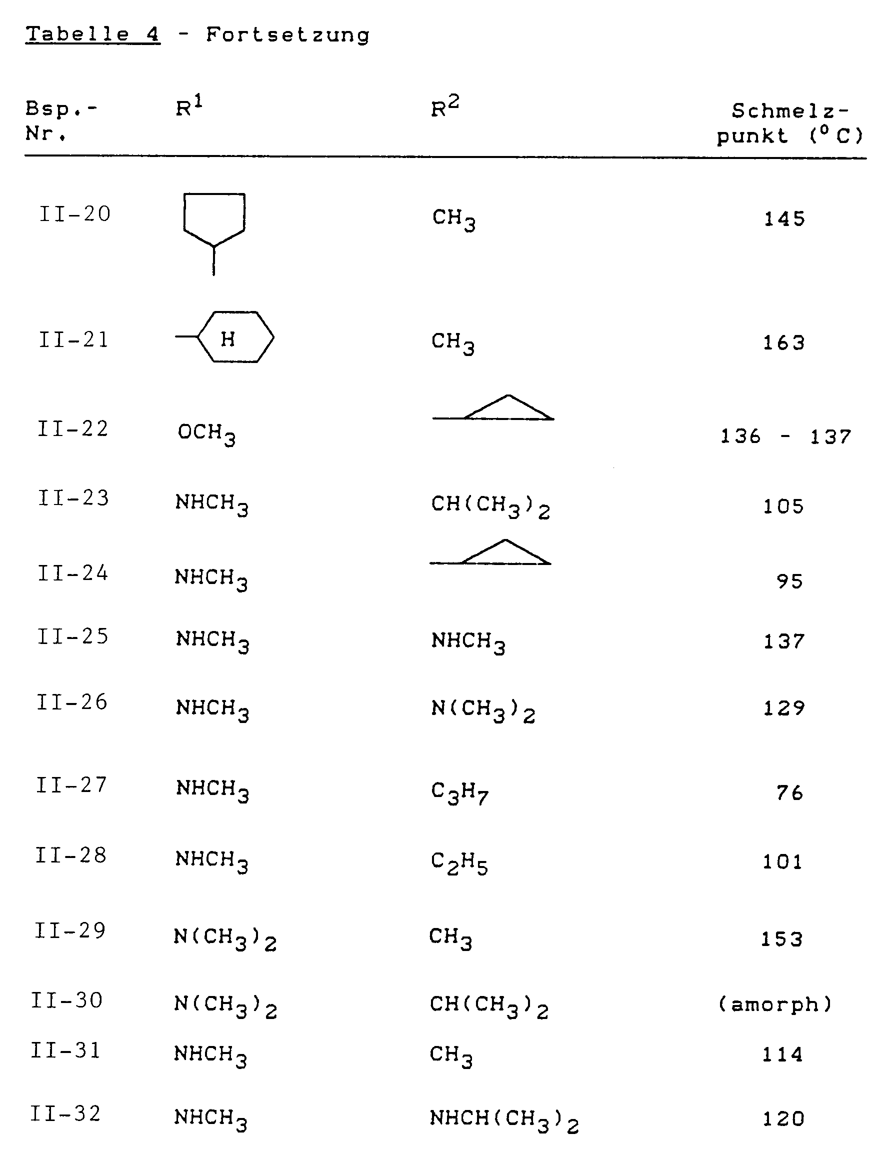

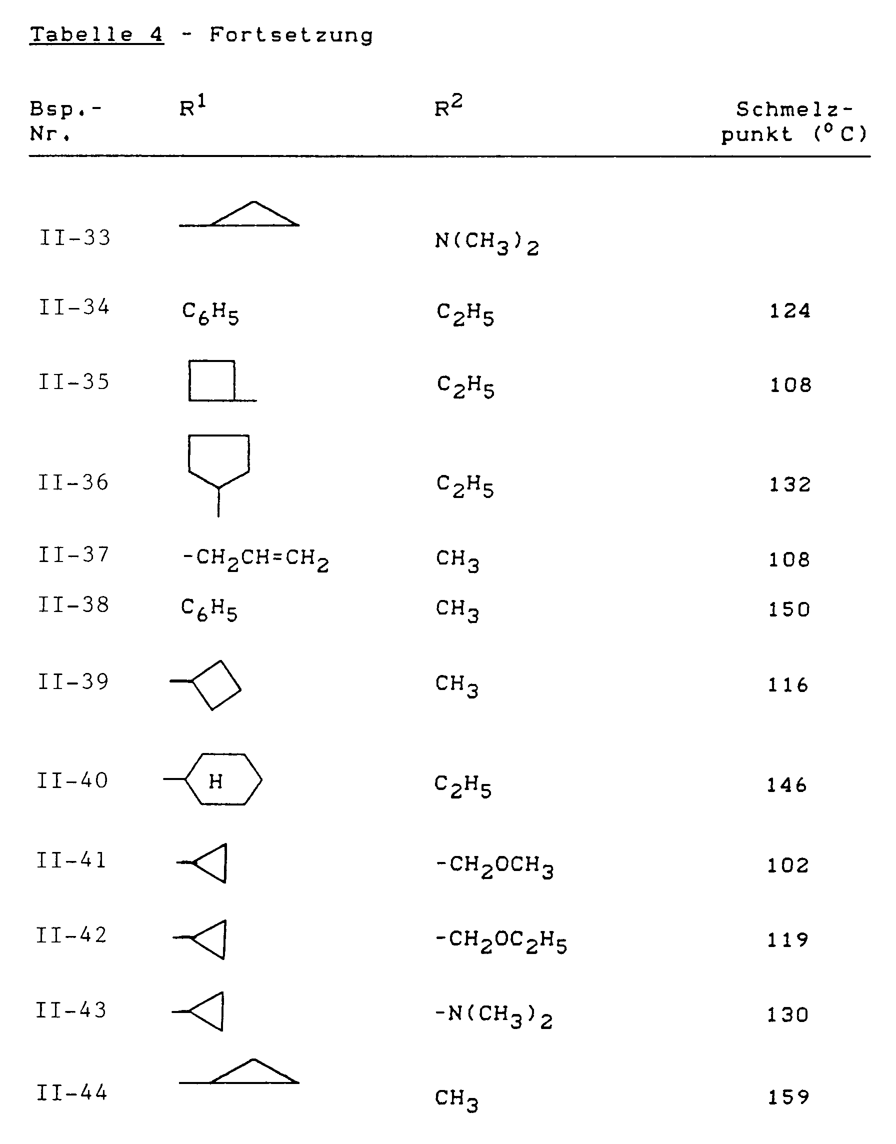

Beispiele für Hydrazinderivate der Formel (IX), welche analog Beispiel (II-1) - Stufe 1 hergestellt werden können, sind in der nachstehenden Tabelle 5 aufgeführt.

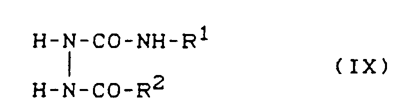

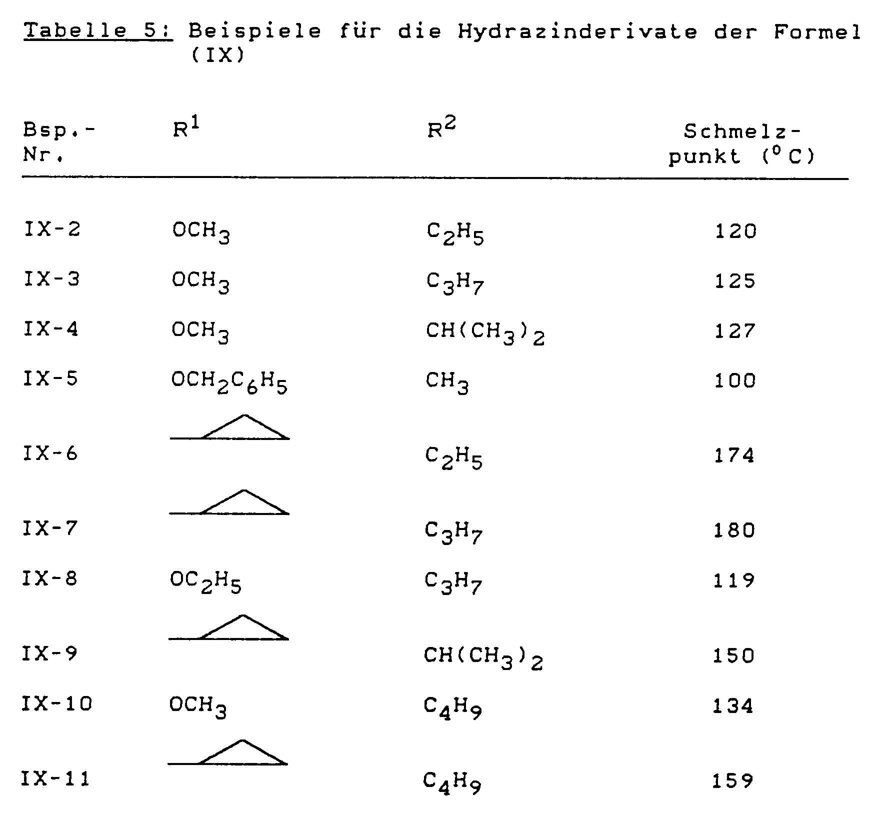

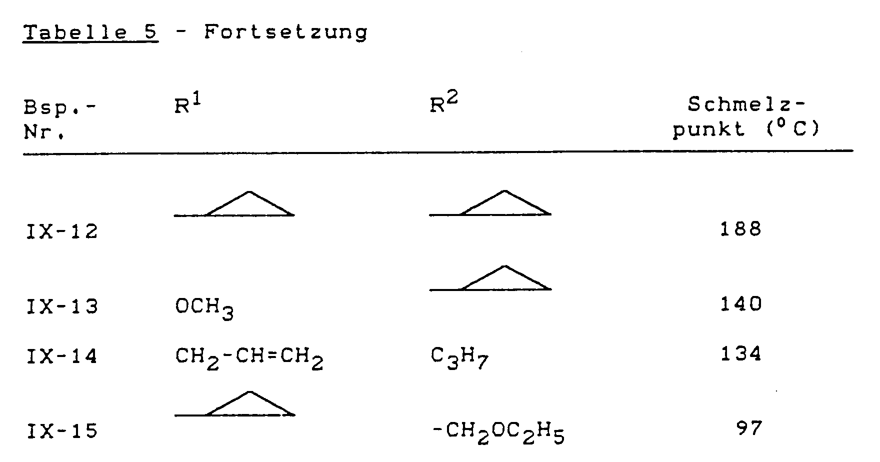

### Ausgangsstoffe der Formel (IV):

### Referenzbeispiel [vgl. EP-A2-0 422 469; Seite 60]

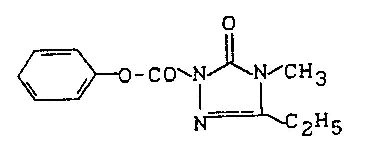

6,4 g (0,05 Mol) 5-Ethyl-4-methyl-2,4-dihydro-3H-1,2,4-triazol-3-on werden in 80 ml Tetrahydrofuran gelöst und unter Stickstoff werden 1,8 g (0,06 Mol) Natriumhydrid (80%ig) dazugegeben. Nach einer Stunde Rühren bei 20°C werden 7,9 g (0,05 Mol) Chlorameisensäurephenylester zugetropft und das Reaktionsgemisch wird noch 20 Stunden bei 20°C gerührt. Nach Einengen wird der Rückstand in Methylenchlorid aufgenommen, mit Wasser gewaschen, mit Natriumsulfat getrocknet und filtriert. Das Filtrat wird eingeengt, der Rückstand mit Diethylether verrieben und das kristallin angefallene Produkt durch Absaugen isoliert.

Man erhält 4,5 g (36% der Theorie) 5-Ethyl-4-methyl-2-phenoxycarbonyl-2,4-dihydro-3H-1,2,4-trizaol-3-on vom Schmelzpunkt 141°C.

Analog Referenzbeispiel können beispielsweise auch die in der nachstehenden Tabelle 6 aufgeführten Verbindungen der Formel (IV) hergestellt werden.

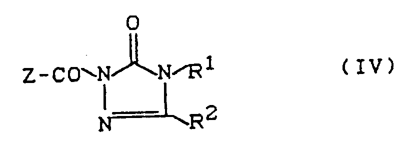

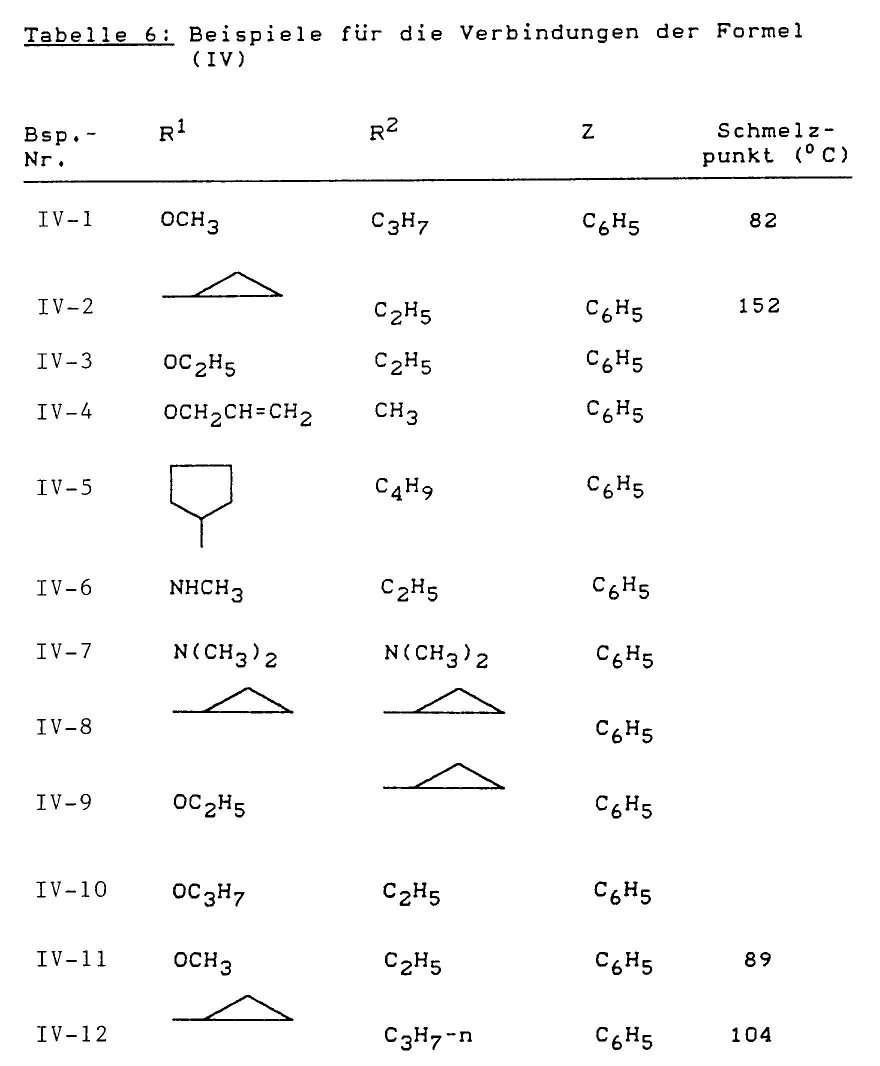

### Anwendungsbeispiele:

Bei den folgenden Anwendungsbeispielen wird das bekannte Herbizid Isocarbamid nachstehender Formel (A) als Vergleichssubstanz herangezogen: 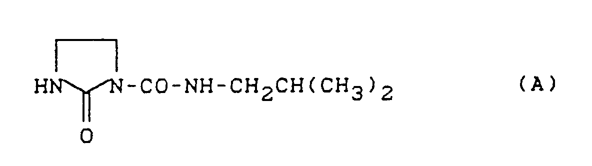
Die Formeln der für die Anwendungsbeispiele herangezogenen erfindungsgemäßen Verbindungen sind - mit der Numerierung der Herstellungsbeispiele - nachstehend einzeln aufgeführt:

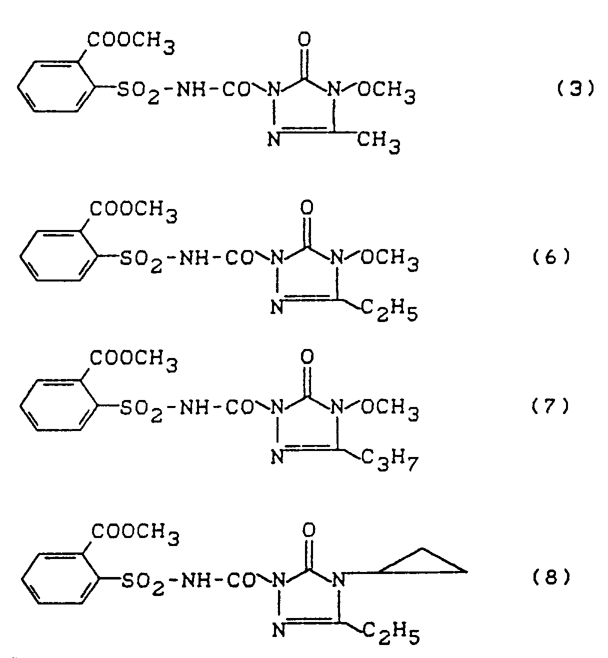

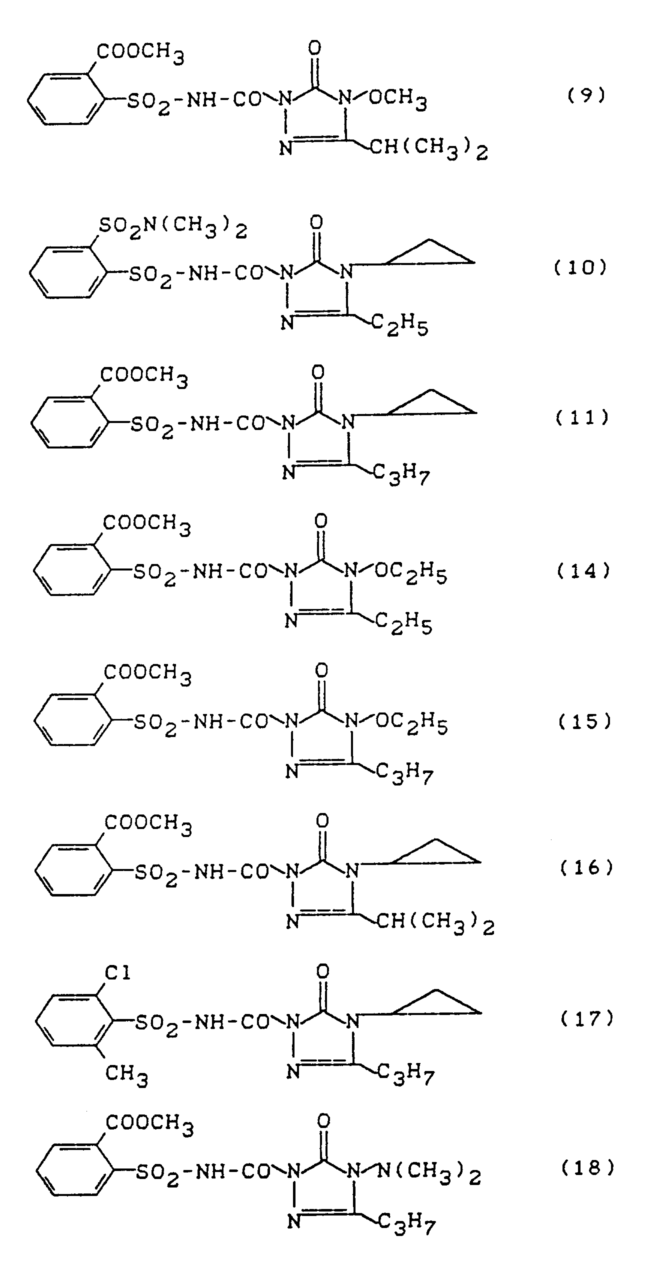

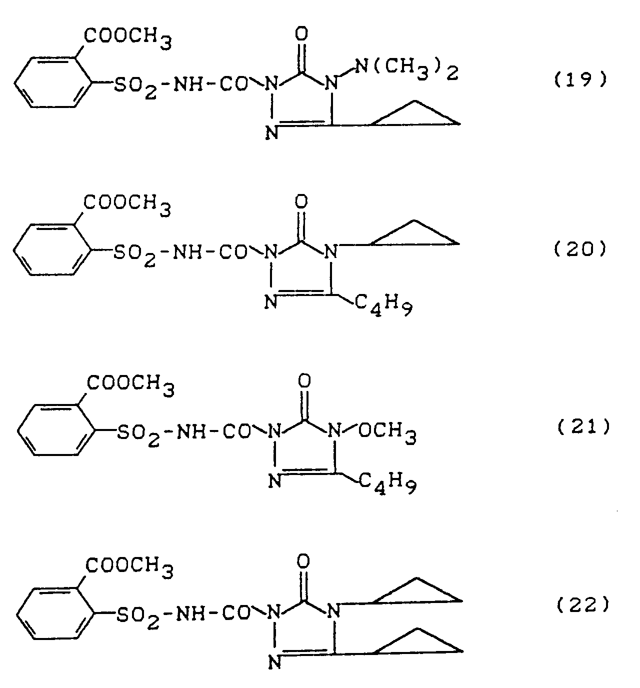

### Beispiel A

### Pre-emergence-Test

- Lösungsmittel:: 5 Gewichtsteile Aceton
- Emulgator:: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100 % = totale Vernichtung
Eine deutliche Überlegenheit in der Wirksamkeit gegenüber dem Stand der Technik zeigen in diesem Test z.B. die Verbindungen gemäß den Herstellungsbeispielen
3, 6, 7, 8, 9, 10, 11, 14, 15, 16, 17 und 22.

### Beispiel B

### Post-emergence-Test

- Lösungsmittel:: 5 Gewichtsteile Aceton
- Emulgator:: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
O % = keine Wirkung (wie unbehandelte Kontrolle)
100 % = totale Vernichtung
Eine deutliche Überlegenheit in der Wirksamkeit gegenüber dem Stand der Technik zeigen in diesem Test z.B. die Verbindungen gemäß den Herstellungsbeispielen
6, 7, 8, 9, 10, 11, 14, 15, 16, 17, 18, 20 und 22.

### Beispiel C

### Pyricularia-Test (Reis) /protektiv

- Lösungsmittel:: 12,5 Gewichtsteile Aceton
- Emulgator:: 0,3 Gewichtsteile Alkylarylpolyglykolether
Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine gute Wirkung zeigen in diesem Test z.B. die Verbindungen gemäß folgender Herstellungsbeispiele:
3, 6, 7, 18, 19, 20 und 21.

### Beispiel D

### Pyricularia-Test (Reis)/systemisch

- Lösungsmittel:: 12,5 Gewichtsteile Aceton
- Emulgator:: 0,3 Gewichtsteile Alkylarylpolyglykolether
Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegeben Menge Emulgator auf die gewünschte Konzentration.

Zur Prüfung aus systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine ausgezeichnete Wirksamkeit zeigen in diesem Test z.B. die Verbindungen gemäß folgender Herstellungsbeispiele: 3, 6, 7, 18, 19, 20 und 21.

## Patentansprüche (Patentansprüche für folgende(n) Vertragsstaat(en): BE, CH, DE, DK, FR, GB, IT, LI, NL)

1. Sulfonylaminocarbonyltriazolinone der allgemeinen Formel (I), 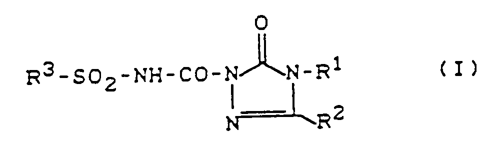 in welcher
R¹ für Allyl, für C₃-C₆-Cycloalkyl, für Phenyl, für Benzyl, für C₁-C₃-Alkoxy, für C₁-C₃-Alkylamino oder für Di-(C₁-C₂-alkyl)-amino steht,
R² für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
R³ für die Gruppierung 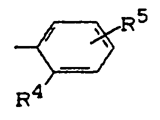 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
R⁵ für Wasserstoff, Fluor, Chlor oder Brom steht;
weiterhin
R³ für den Rest 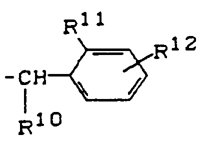 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 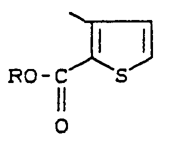 steht,
worin R für C₁-C₄-Alkyl steht, oder
für den Rest 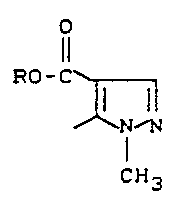 steht,
worin R für C₁-C₄-Alkyl steht,
sowie Salze von Verbindungen der Formel (I),
ausgenommen jedoch - für die Vertragsstaaten BE, CH, DE, FR, GB, IT, LI und NL - die Verbindungen:
4-Cyclopropyl-5-methyl-2-(2-methoxycarbonyl-phenylsulfonyl-aminocarbonyl)-2,4-dihydro-3H-1,2,4-triazol-3-on
und
4-Dimethylamino-5-methyl-2-(2-methoxycarbonyl-phenylsulfonyl-aminocarbonyl)-2,4-dihydro-3H-1,2,4-triazol-3-on.

2. Sulfonylaminocarbonyltriazolinone der Formel (I) gemäß Anspruch 1, dadurch gekennzeichnet, daß darin
R¹ für C₁-C₃-Alkoxy steht,
R² für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
R³ für die Gruppierung 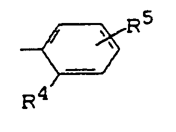 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
R⁵ für Wasserstoff, Fluor, Chlor oder Brom steht;
weiterhin
R³ für den Rest 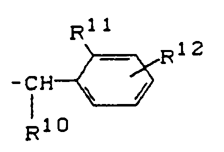 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 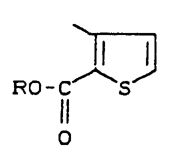 steht,
worin R für C₁-C₄-Alkyl steht, oder
für den Rest 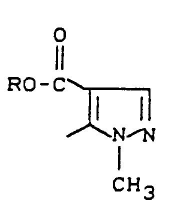 steht,
worin R für C₁-C₄-Alkyl steht,
sowie Salze dieser Verbindungen.

3. Sulfonylaminocarbonyltriazolinone der Formel (I) gemäß Anspruch 1, dadurch gekennzeichnet, daß darin
R¹ für C₁-C₃-Alkoxy steht,
R² für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
R³ für die Gruppierung 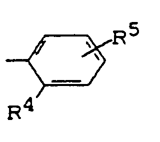 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
R⁵ für Wasserstoff, Fluor, Chlor oder Brom steht; oder
weiterhin
R³ für den Rest 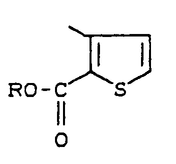 steht,
worin R für C₁-C₄-Alkyl steht,
sowie Salze dieser Verbindungen.

4. Verfahren zur Herstellung von Sulfonylaminocarbonyltriazolinonen der allgemeinen Formel (I) gemäß Anspruch 1, dadurch gekennzeichnet, daß man
a) Triazolinone der allgemeinen Formel (II), 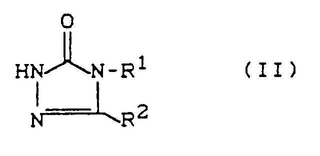 in welcher
R¹ und R² die in Anspruch 1 angegebenen Bedeutungen haben,
mit Sulfonylisocyanaten der allgemeinen Formel (III),
R³-SO₂-N=C=O (III)
in welcher
R³ die in Anspruch 1 angegebene Bedeutung hat,
gegebenenfalls in Gegenwart eines Verdünnungsmittels umsetzt, oder daß man
b) Triazolinon-Derivate der allgemeinen Formel (IV), 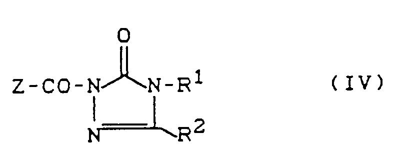 in welcher
R¹ und R² die in Anspruch 1 angegebenen Bedeutungen haben und
Z für Chlor, C₁-C₄-Alkoxy, Benzyloxy oder Phenoxy steht,
mit Sulfonsäureamiden der allgemeinen Formel (V),
R³-SO₂-NH₂ (V)
in welcher
R³ die in Anspruch 1 angegebene Bedeutung hat,
gegebenenfalls in Gegenwart eines Säureakzeptors und gegebenenfalls in Gegenwart eines Verdünnungsmittels umsetzt, oder daß man
c) Triazolinone der allgemeinen Formel (II), 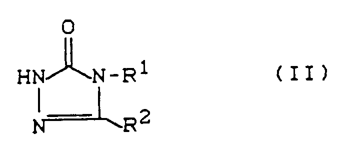 in welcher
R¹ und R² die in Anspruch 1 angegebenen Bedeutungen haben,
mit Sulfonsäureamid-Derivaten der allgemeinen Formel (VI),
R³-SO₂-NH-CO-Z (VI)
in welcher
R³ die in Anspruch 1 angegebene Bedeutung hat und
Z für Chlor, C₁-C₄-Alkoxy, Benzyloxy oder Phenoxy steht,
gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

5. Herbizide Mittel, gekennzeichnet durch einen Gehalt an mindestens einem Sulfonylaminocarbonyltriazolinon der Formel (I) gemäß Anspruch 1.

6. Verwendung von Sulfonylaminocarbonyltriazolinonen der allgemeinen Formel (I) gemäß Anspruch 1 zur Bekämpfung von unerwünschtem Pflanzenwachstum.

7. Verfahren zur Bekämpfung von Unkräutern, dadurch gekennzeichnet, daß man Sulfonylaminocarbonyltriazolinone der allgemeinen Formel (I) gemäß Anspruch 1 auf die Unkräuter oder ihren Lebensraum einwirken läßt.

8. Verfahren zur Herstellung von herbiziden Mitteln, dadurch gekennzeichnet, daß man Sulfonylaminocarbonyltriazolinone der allgemeinen Formel (I) gemäß Anspruch 1 mit Streckmitteln und/oder oberflächenaktiven Mitteln vermischt.

## Patentansprüche (Patentansprüche für folgende(n) Vertragsstaat(en): ES)

1. Verfahren zur Herstellung von Sulfonylaminocarbonyltriazolinonen der allgemeinen Formel (I), 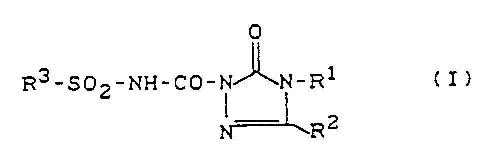 in welcher
R¹ für Allyl, für C₃-C₆-Cycloalkyl, für Phenyl, für Benzyl, für C₁-C₃-Alkoxy, für C₁-C₃-Alkylamino oder für Di-(C₁-C₂-alkyl)-amino steht,
R² für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
R³ für die Gruppierung 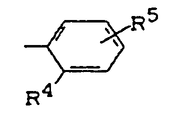 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
R⁵ für Wasserstoff, Fluor, Chlor oder Brom steht;
weiterhin
R³ für den Rest 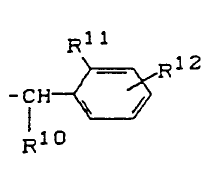 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 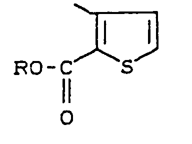 steht,
worin R für C₁-C₄-Alkyl steht, oder
für den Rest 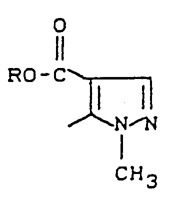 steht,
worin R für C₁-C₄-Alkyl steht,
sowie von Salzen der Verbindungen der Formel (I), dadurch gekennzeichnet, daß man
a) Triazolinone der allgemeinen Formel (II), 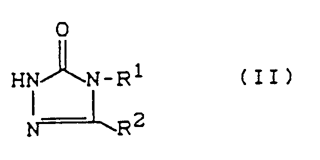 in welcher
R¹ und R² die oben (bei Formel I)) angegebenen Bedeutungen haben,
mit Sulfonylisocyanaten der allgemeinen Formel (III),
R³-SO₂-N=C=O (III)
in welcher
R³ die oben angegebene Bedeutung hat,
gegebenenfalls in Gegenwart eines Verdünnungsmittels umsetzt, oder daß man
b) Triazolinon-Derivate der allgemeinen Formel (IV), 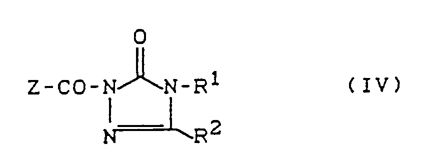 in welcher
R¹ und R² die oben angegebenen Bedeutungen haben und
Z für Chlor, C₁-C₄-Alkoxy, Benzyloxy oder Phenoxy steht,
mit Sulfonsäureamiden der allgemeinen Formel (V),
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt, oder daß man
c) Triazolinone der allgemeinen Formel (II), 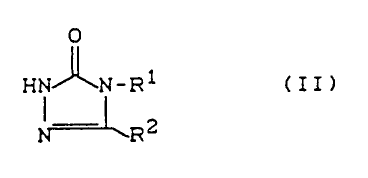 in welcher
R¹ und R² die oben angegebenen Bedeutungen haben,
mit Sulfonsäureamid-Derivaten der allgemeinen Formel (VI),
R³-SO₂-NH-CO-Z (VI)
in welcher
R³ die oben angegebene Bedeutung hat und
Z für Chlor, C₁-C₄-Alkoxy, Benzyloxy oder Phenoxy steht,
gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

2. Herbizide Mittel, gekennzeichnet durch einen Gehalt an mindestens einem Sulfonylaminocarbonyltriazolinon der Formel (I) gemäß Anspruch 1.

3. Verwendung von Sulfonylaminocarbonyltriazolinonen der allgemeinen Formel (I) gemäß Anspruch 1 zur Bekämpfung von unerwünschtem Pflanzenwachstum.

4. Verfahren zur Bekämpfung von Unkräutern, dadurch gekennzeichnet, daß man Sulfonylaminocarbonyltriazolinone der allgemeinen Formel (I) gemäß Anspruch 1 auf die Unkräuter oder ihren Lebensraum einwirken läßt.

5. Verfahren zur Herstellung von herbiziden Mitteln, dadurch gekennzeichnet, daß man Sulfonylaminocarbonyltriazolinone der allgemeinen Formel (I) gemäß Anspruch 1 mit Streckmitteln und/oder oberflächenaktiven Mitteln vermischt.

## Claims (Claims for the following Contracting State(s): BE, CH, DE, DK, FR, GB, IT, LI, NL)

1. Sulphonylaminocarbonyltriazolinones of the general formula (I) 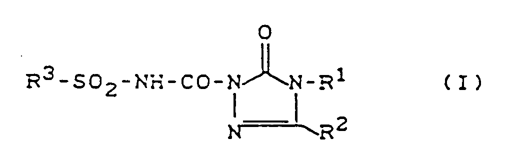 in which
R¹ represents allyl, C₃-C₆-cycloalkyl, phenyl, benzyl, C₁-C₃-alkoxy, C₁-C₃-alkylamino or di-(C₁-C₂-alkyl)-amino,
R² represents C₁-C₄-alkyl which is optionally substituted by fluorine and/or chlorine, methoxy or ethoxy, or represents C₃-C₆-cycloalkyl, phenyl, C₁-C₃-alkoxy, C₁-C₃-alkylamino or di-(C₁-C₂-alkyl)-amino, and
R³ represents the group 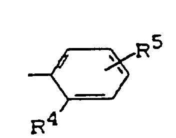 in which
R⁴ represents fluorine, chlorine, bromine, methyl, trifluoromethyl, methoxy, difluoromethoxy, trifluoromethoxy, 2-chloroethoxy, 2-methoxyethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, phenyl, phenoxy or C₁-C₃-alkoxycarbonyl and
R⁵ represents hydrogen, fluorine, chlorine or bromine;
furthermore
R³ represents the radical 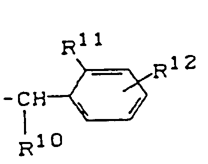 in which,
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 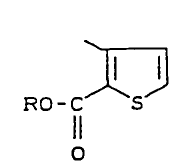 in which R represents C₁-C₄-alkyl, or
represents the radical 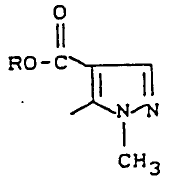 in which R represents C₁-C₄-alkyl,
and salts of compounds of the formula (I),
but with the exception - for the contracting states BE, CH, DE, FR, GB, IT, LI and NL - of the compounds:
4-cyclopropyl-5-methyl-2-(2-methoxycarbonyl-phenylsulphonyl-aminocarbonyl)-2,4-dihydro-3H-1,2,4-triazol-3-one
and
4-dimethylamino-5-methyl-2-(2-methoxycarbonyl-phenylsulphonyl-aminocarbonyl)-2,4-dihydro-3H-1,2,4-triazol-3-one.

2. Sulphonylaminocarbonyltriazolinones of the formula (I) according to Claim 1, characterized in that, in this formula,
R¹ represents C₁-C₃-alkoxy,
R² represents C₁-C₄-alkyl which is optionally substituted by fluorine and/or chlorine, methoxy or ethoxy, or represents C₃-C₆-cycloalkyl, phenyl, C₁-C₃-alkoxy, C₁-C₃-alkylamino or di-(C₁-C₂-alkyl)-amino, and
R³ represents the group 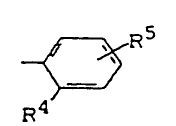 in which
R⁴ represents fluorine, chlorine, bromine, methyl, trifluoromethyl, methoxy, difluoromethoxy, trifluoromethoxy, 2-chloroethoxy, 2-methoxyethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, phenyl, phenoxy or C₁-C₃-alkoxycarbonyl and
R⁵ represents hydrogen, fluorine, chlorine or bromine;
furthermore
R³ represents the radical 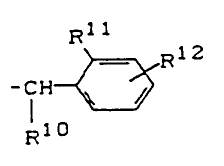 in which,
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 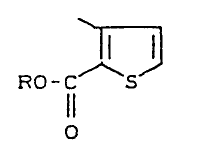 in which R represents C₁-C₄-alkyl, or
represents the radical 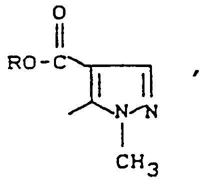 in which R represents C₁-C₄-alkyl,
and salts of these compounds.

3. Sulphonylaminocarbonyltriazolinones of the formula (I) according to Claim 1, characterized in that, in this formula,
R¹ represents C₁-C₃-alkoxy,
R² represents C₁-C₄-alkyl which is optionally substituted by fluorine and/or chlorine, methoxy or ethoxy, or represents C₃-C₆-cycloalkyl, phenyl, C₁-C₃-alkoxy, C₁-C₃-alkylamino or di-(C₁-C₂-alkyl)-amino, and
R³ represents the group 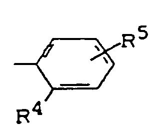 in which
R⁴ represents fluorine, chlorine, bromine, methyl, trifluoromethyl, methoxy, difluoromethoxy, trifluoromethoxy, 2-chloroethoxy, 2-methoxyethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, phenyl, phenoxy or C₁-C₃-alkoxycarbonyl and
R⁵ represents hydrogen, fluorine, chlorine or bromine;
or
furthermore
R³ represents the radical 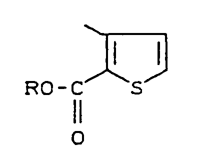 in which R represents C₁-C₄-alkyl,
and salts of these compounds.

4. Process for the preparation of sulphonylaminocarbonyltriazolinones of the general formula (I) according to Claim 1, characterized in that
a) triazolinones of the general formula (II), 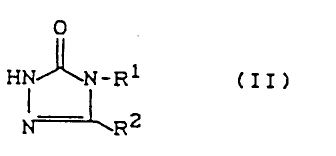 in which
R¹ and R² have the meanings given in Claim 1,
are reacted with sulphonyl isocyanates of the general formula (III),
R³-SO₂-N=C=O (III)
in which
R³ has the meaning given in Claim 1,
if appropriate in the presence of a diluent, or in that
b) triazolinone derivatives of the general formula (IV), 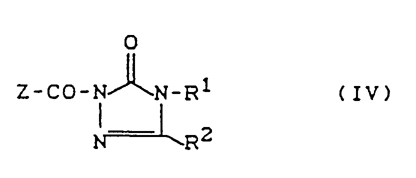 in which
R¹ and R² have the meanings given in Claim 1 and
Z represents chlorine, C₁-C₄-alkoxy, benzyloxy or phenoxy,
are reacted with sulphonamides of the general formula (V),
R³-SO₂-NH₂ (V)
in which
R³ has the meaning given in Claim 1,
if appropriate in the presence of an acid acceptor and if appropriate in the presence of a diluent, or in that,
c) triazolinones of the general formula (II), 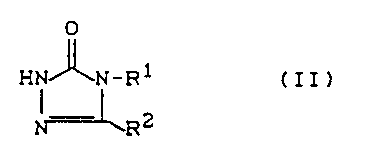 in which
R¹ and R² have the meanings given in Claim 1,
are reacted with sulphonamide derivatives of the general formula (VI),
R³-SO₂-NH-CO-Z (VI)
in which
R³ has the meaning given in Claim 1 and
Z represents chlorine, C₁-C₄-alkoxy, benzyloxy or phenoxy,
if appropriate in the presence of an acid acceptor and if appropriate in the presence of a diluent, and, if appropriate, salts are prepared by customary methods from the compounds of the formula (I) which have been prepared by process (a), (b) or (c).

5. Herbicidal compositions, characterized in that they comprise at least one sulphonylaminocarbonyltriazolinone of the formula (I) according to Claim 1.

6. Use of sulphonylaminocarbonyltriazolinones of the general formula (I) according to Claim 1 for combating undesirable plant growth.

7. Method of combating weeds, characterized in that sulphonylaminocarbonyltriazolinones of the general formula (I) according to Claim 1 are allowed to act on the weeds or their environment.

8. Process for the preparation of herbicidal compositions, characterized in that sulphonylaminocarbonyltriazolinones of the general formula (I) according to Claim 1 are mixed with extenders and/or surface-active agents.

## Claims (Claims for the following Contracting State(s): ES)

1. Process for the preparation of sulphonylaminocarbonyltriazolinones of the general formula (I), 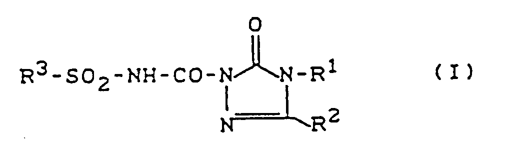 in which
R¹ represents allyl, C₃-C₆-cycloalkyl, phenyl, benzyl, C₁-C₃-alkoxy, C₁-C₃-alkylamino or di-(C₁-C₂-alkyl)-amino,
R² represents C₁-C₄-alkyl which is optionally substituted by fluorine and/or chlorine, methoxy or ethoxy, or represents C₃-C₆-cycloalkyl, phenyl, C₁-C₃-alkoxy, C₁-C₃-alkylamino or di-(C₁-C₂-alkyl)-amino, and
R³ represents the group 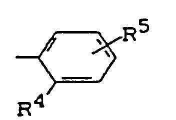 in which
R⁴ represents fluorine, chlorine, bromine, methyl, trifluoromethyl, methoxy, difluoromethoxy, trifluoromethoxy, 2-chloroethoxy, 2-methoxyethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, phenyl, phenoxy or C₁-C₃-alkoxycarbonyl and
R⁵ represents hydrogen, fluorine, chlorine or bromine;
furthermore
R³ represents the radical 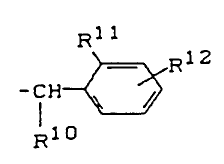 in which,
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 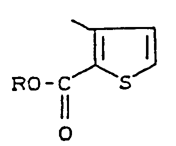 in which R represents C₁-C₄-alkyl, or
represents the radical 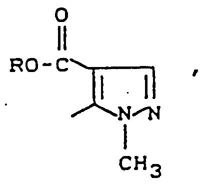 in which R represents C₁-C₄-alkyl,
and of salts of the compounds of the formula (I), characterized in that
a) triazolinones of the general formula (II), 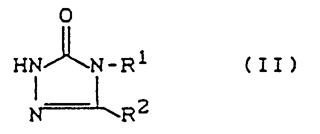 in which
R¹ and R² have the meanings given above (in formula (I)),
are reacted with sulphonyl isocyanates of the general formula (III),
R³-SO₂-N=C=O (III)
in which
R³ has the meaning given above,
if appropriate in the presence of a diluent, or in that
b) triazolinone derivatives of the general formula (IV), 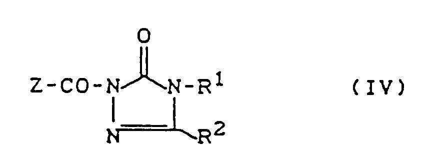 in which
R¹ and R² have the meanings given above and
Z represents chlorine, C₁-C₄-alkoxy, benzyloxy or phenoxy,
are reacted with sulphonamides of the general formula (V),
R³-SO₂-NH₂ (V)
in which
R³ has the meaning given above,
if appropriate in the presence of an acid acceptor and if appropriate in the presence of a diluent, or in that,
c) triazolinones of the general formula (II), 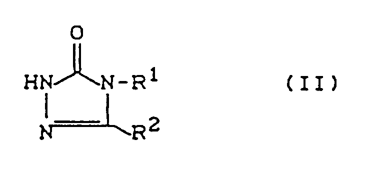 in which
R¹ and R² have the meanings given above,
are reacted with sulphonamide derivatives of the general formula (VI),
R³-SO₂-NH-CO-Z (VI)
in which
R³ has the meaning given above and
Z represents chlorine, C₁-C₄-alkoxy, benzyloxy or phenoxy,
if appropriate in the presence of an acid acceptor and if appropriate in the presence of a diluent, and, if appropriate, salts are prepared by customary methods from the compounds of the formula (I) which have been prepared by process (a), (b) or (c).

2. Herbicidal compositions, characterized in that they comprise at least one sulphonylaminocarbonyltriazolinone of the formula (I) according to Claim 1.

3. Use of sulphonylaminocarbonyltriazolinones of the general formula (I) according to Claim 1 for combating undesirable plant growth.

4. Method of combating weeds, characterized in that sulphonylaminocarbonyltriazolinones of the general formula (I) according to Claim 1 are allowed to act on the weeds or their environment.

5. Process for the preparation of herbicidal compositions, characterized in that sulphonylaminocarbonyltriazolinones of the general formula (I) according to Claim 1 are mixed with extenders and/or surface-active agents.

## Revendications (Revendications pour l'(les) Etat(s) contractant(s) suivant(s): BE, CH, DE, DK, FR, GB, IT, LI, NL)

1. Sulfonylaminocarbonyltriazolinones répondant à la formule générale (I) 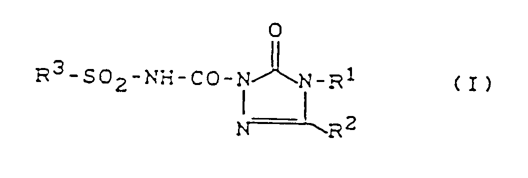 dans laquelle
R¹ représente un groupe allyle, un groupe cycloalkyle en C₃-C₆, un groupe phényle, un groupe benzyle, un groupe alcoxy en C₁-C₃, un groupe alkyl(en C₁-C₃)amino ou un groupe di(alkyl en C₁-C₂)amino;
R² représente un groupe alkyle en C₁-C₄ portant éventuellement un ou plusieurs substituants fluoro et/ou chloro, méthoxy ou éthoxy; un groupe cycloalkyle en C₃-C₆; un groupe phényle; un groupe alcoxy en C₁-C₃; un groupe alkyl(en C₁-C₃)amino; ou encore un groupe di(alkyl en C₁-C₂)amino; et
R³ représente le groupement 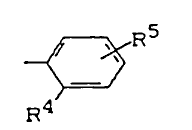 dans lequel
R⁴ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloréthoxy, un groupe 2-méthoxyéthoxy, un groupe alkyl(en C₁-C₃)thio, un groupe alkyl(en C₁-C₃)sulfinyle, un groupe alkyl(en C₁-C₃)-sulfonyle, un groupe diméthylaminosulfonyle, un groupe diéthylaminosulfonyle, un groupe N-méthoxy-N-méthylaminosulfonyle, un groupe phényle, un groupe phénoxy ou un groupe alcoxy(en C₁-C₃)-carbonyle; et
R⁵ représente un atome d'hydrogène, un atome de fluor, un atome de chlore ou un atome de brome;
en outre,
R³ représente le radical 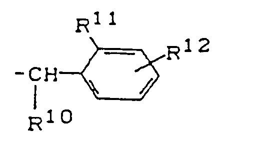 dans lequel
R¹⁰ représente un atome d'hydrogène;
R¹¹ représente un atome de fluor, un atome de chlore, un atome de brome, un groupe méthyle, un groupe méthoxy, un groupe difluorométhoxy, un groupe trifluorométhoxy, un groupe éthoxy, un groupe méthoxycarbonyle, un groupe éthoxycarbonyle, un groupe méthylsulfonyle ou un groupe diméthylaminosulfonyle; et
R¹² représente un atome d'hydrogène;
en outre,
R³ représente le radical 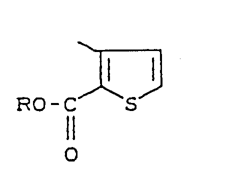 dans lequel R représente un groupe alkyle en C₁-C₄, ou
représente le radical 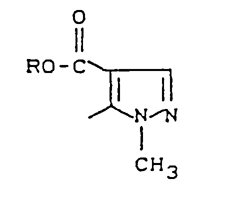 dans lequel R représente un groupe alkyle en C₁-C₄,
ainsi que les sels des composés de formule (I),
à l'exception toutefois - pour les Etats contractants : BE, CH, DE, FR, GB, IT, LI et NL - des composés :
la 4-cyclopropyl-5-méthyl-2-(2-méthoxycarbonylphénylsulfonylaminocarbonyl)-2,4-dihydro-3H-1,2,4-triazol-3-one
et
la 4-diméthylamino-5-méthyl-2-(2-méthoxycarbonylphénylsulfonylaminocarbonyl)-2,4-dihydro-3H-1,2,4-triazol-3-one.

2. Sulfonylaminocarbonyltriazolinones de formule (I) selon la revendication 1, caractérisées en ce que, dans ces composés,
R¹ représente un groupe alcoxy en C₁-C₃;
R² représente un groupe alkyle en C₁-C₄ portant éventuellement un ou plusieurs substituants fluoro et/ou chloro, méthoxy ou éthoxy; un groupe cycloalkyle en C₃-C₆; un groupe phényle; un groupe alcoxy en C₁-C₃; un groupe alkyl(en C₁-C₃)amino; ou encore un groupe di(alkyl en C₁-C₂)amino; et
R³ représente le groupement 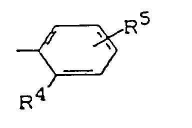 dans lequel
R⁴ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloréthoxy, un groupe 2-méthoxyéthoxy, un groupe alkyl(en C₁-C₃)thio, un groupe alkyl(en C₁-C₃)sulfinyle, un groupe alkyl(en C₁-C₃)-sulfonyle, un groupe diméthylaminosulfonyle, un groupe diéthylaminosulfonyle, un groupe N-méthoxy-N-méthylaminosulfonyle, un groupe phényle, un groupe phénoxy ou un groupe alcoxy(en C₁-C₃)-carbonyle; et
R⁵ représente un atome d'hydrogène, un atome de fluor, un atome de chlore ou un atome de brome;
en outre,
R³ représente le radical 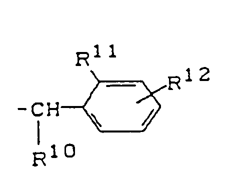 dans lequel
R¹⁰ représente un atome d'hydrogène;
R¹¹ représente un atome de fluor, un atome de chlore, un atome de brome, un groupe méthyle, un groupe méthoxy, un groupe difluorométhoxy, un groupe trifluorométhoxy, un groupe éthoxy, un groupe méthoxycarbonyle, un groupe éthoxycarbonyle, un groupe méthylsulfonyle ou un groupe diméthylaminosulfonyle; et
R¹² représente un atome d'hydrogène;
en outre,
R³ représente le radical 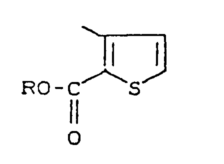 dans lequel R représente un groupe alkyle en C₁-C₄, ou
représente le radical 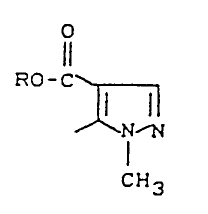 dans lequel R représente un groupe alkyle en C₁-C₄,
ainsi que des sels de ces composés.

3. Sulfonylaminocarbonyltriazolinones de formule (I) selon la revendication 1, caractérisées en ce que, dans ces composés,
R¹ représente un groupe alcoxy en C₁-C₃;
R² représente un groupe alkyle en C₁-C₄ portant éventuellement un ou plusieurs substituants fluoro et/ou chloro, méthoxy ou éthoxy; un groupe cycloalkyle en C₃-C₆; un groupe phényle; un groupe alcoxy en C₁-C₃; un groupe alkyl(en C₁-C₃)amino; ou encore un groupe di(alkyl en C₁-C₂)amino; et
R³ représente le groupement 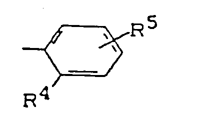 dans lequel
R⁴ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloréthoxy, un groupe 2-méthoxyéthoxy, un groupe alkyl(en C₁-C₃)thio, un groupe alkyl(en C₁-C₃)sulfinyle, un groupe alkyl(en C₁-C₃)-sulfonyle, un groupe diméthylaminosulfonyle, un groupe diéthylaminosulfonyle, un groupe N-méthoxy-N-méthylaminosulfonyle, un groupe phényle, un groupe phénoxy ou un groupe alcoxy(en C₁-C₃)-carbonyle; et
R⁵ représente un atome d'hydrogène, un atome de fluor, un atome de chlore ou un atome de brome;
en outre,
R³ représente le radical 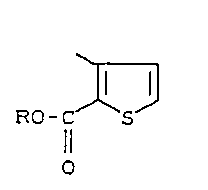 dans lequel R représente un groupe alkyle en C₁-C₄,
ainsi que des sels de ces composés.

4. Procédé pour la préparation de sulfonylaminocarbonyltriazolinones répondant à la formule générale (I) selon la revendication 1, caractérisé en ce qu'on fait réagir
a) des triazolinones répondant à la formule générale (II) 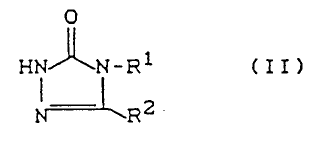 dans laquelle
R¹ et R² ont les significations indiquées à la revendication 1,
avec des sulfonylisocyanates répondant à la formule générale (III)
R³-SO₂-N=C=O (III)
dans laquelle
R³ a la signification indiquée à la revendication 1,
éventuellement en présence d'un diluant, ou bien en ce qu'on fait réagir
b) des dérivés de triazolinones, répondant à la formule générale (IV) 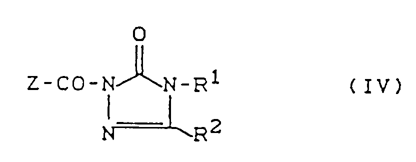 dans laquelle
R¹ et R² ont les significations indiquées à la revendication 1, et
Z représente un atome de chlore, un groupe alcoxy en C₁-C₄, un groupe benzyloxy ou un groupe phénoxy,
avec des amides d'acides sulfoniques répondant à la formule générale (V)
R³-SO₂-NH₂ (V)
dans laquelle
R³ a la signification indiquée à la revendication 1,
éventuellement en présence d'un agent neutralisateur d'acides et éventuellement en présence d'un diluant, ou bien en ce qu'on fait réagir
c) des triazolinones répondant à la formule générale (II) 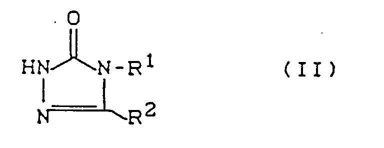 dans laquelle
R¹ et R² ont les significations indiquées à la revendication 1,
avec des dérivés d'amides d'acides sulfoniques répondant à la formule générale (VI)
R³-SO₂-NH-CO-Z (VI)
dans laquelle
R³ a la signification indiquée à la revendication 1, et
Z représente un atome de chlore, un groupe alcoxy en C₁-C₄, un groupe benzyloxy ou un groupe phénoxy,
éventuellement en présence d'un agent neutralisateur d'acides et éventuellement en présence d'un diluant, et, le cas échéant, on forme des sels à partir des composés de formule (I) préparés conformément aux procédés (a), (b) ou (c), conformément à des procédés habituels.

5. Agents herbicides caractérisés par une teneur en au moins une sulfonylaminocarbonyltriazolinone de formule (I) selon la revendication 1.

6. Utilisation de sulfonylaminocarbonyltriazolinones répondant à la formule générale (I) selon la revendication 1, pour lutter contre la croissance non désirée de plantes.

7. Procédé pour lutter contre les mauvaises herbes, caractérisé en ce qu'on laisse agir des sulfonylaminocarbonyltriazolinones répondant à la formule générale (I) selon la revendication 1, sur les mauvaises herbes ou sur leur biotope.

8. Procédé pour la préparation d'agents herbicides, caractérisé en ce qu'on mélange des sulfonylaminocarbonyltriazolinones répondant à la formule générale (I) selon la revendication 1, avec des diluants et/ou des agents tensioactifs.

## Revendications (Revendications pour l'(les) Etat(s) contractant(s) suivant(s): ES)

1. Procédé pour la préparation de sulfonylaminocarbonyltriazolinones répondant à la formule générale (I) 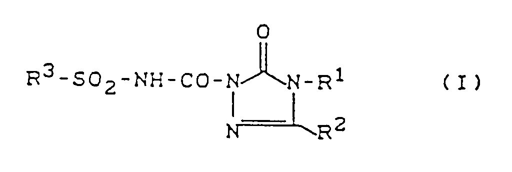 dans laquelle
R¹ représente un groupe allyle, un groupe cycloalkyle en C₃-C₆, un groupe phényle, un groupe benzyle, un groupe alcoxy en C₁-C₃, un groupe alkyl(en C₁-C₃)amino ou un groupe di(alkyl en C₁-C₂)amino;
R² représente un groupe alkyle en C₁-C₄ portant éventuellement un ou plusieurs substituants fluoro et/ou chloro, méthoxy ou éthoxy; un groupe cycloalkyle en C₃-C₆; un groupe phényle; un groupe alcoxy en C₁-C₃; un groupe alkyl(en C₁-C₃)amino; ou encore un groupe di(alkyl en C₁-C₂)amino; et
R³ représente le groupement 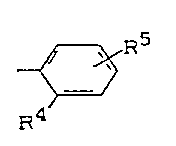 dans lequel
R⁴ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloréthoxy, un groupe 2-méthoxyéthoxy, un groupe alkyl(en C₁-C₃)thio, un groupe alkyl(en C₁-C₃)sulfinyle, un groupe alkyl(en C₁-C₃)-sulfonyle, un groupe diméthylaminosulfonyle, un groupe diéthylaminosulfonyle, un groupe N-méthoxy-N-méthylaminosulfonyle, un groupe phényle, un groupe phénoxy ou un groupe alcoxy(en C₁-C₃)-carbonyle; et
R⁵ représente un atome d'hydrogène, un atome de fluor, un atome de chlore ou un atome de brome;
en outre,
R³ représente le radical 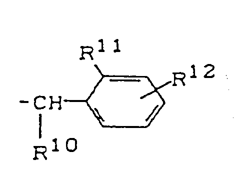 dans lequel
R¹⁰ représente un atome d'hydrogène;
R¹¹ représente un atome de fluor, un atome de chlore, un atome de brome, un groupe méthyle, un groupe méthoxy, un groupe difluorométhoxy, un groupe trifluorométhoxy, un groupe éthoxy, un groupe méthoxycarbonyle, un groupe éthoxycarbonyle, un groupe méthylsulfonyle ou un groupe diméthylaminosulfonyle; et
R¹² représente un atome d'hydrogène;
en outre,
R³ représente le radical 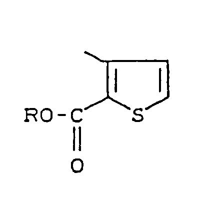 dans lequel R représente un groupe alkyle en C₁-C₄, ou
représente le radical 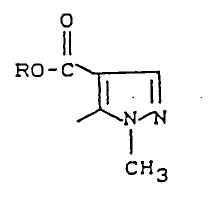 dans lequel R représente un groupe alkyle en C₁-C₄,
ainsi que des sels des composés de formule (I), caractérisé en ce qu'on fait réagir
a) des triazolinones répondant à la formule générale (II) 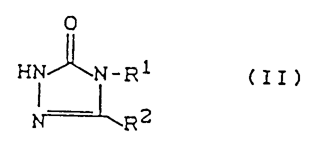 dans laquelle
R¹ et R² ont les significations indiquées ci-dessus (dans la formule I),
avec des sulfonylisocyanates répondant à la formule générale (III)
R³-SO₂-N=C=O (III)
dans laquelle
R³ a la signification indiquée ci-dessus,
éventuellement en présence d'un diluant, ou bien en ce qu'on fait réagir
b) des dérivés de triazolinones, répondant à la formule générale (IV) 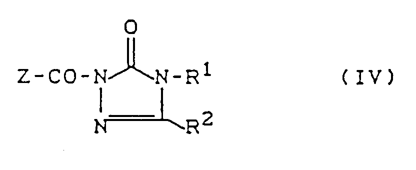 dans laquelle
R¹ et R² ont les significations indiquées ci-dessus, et
Z représente un atome de chlore, un groupe alcoxy en C₁-C₄, un groupe benzyloxy ou un groupe phénoxy,
avec des amides d'acides sulfoniques répondant à la formule générale (V)
R³-SO₂-NH₂ (V)
dans laquelle
R³ a la signification indiquée ci-dessus,
éventuellement en présence d'un agent neutralisateur d'acides et éventuellement en présence d'un diluant, ou bien en ce qu'on fait réagir
c) des triazolinones répondant à la formule générale (II) 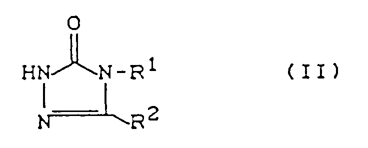 dans laquelle
R¹ et R² ont les significations indiquées ci-dessus,
avec des dérivés d'amides d'acides sulfoniques répondant à la formule générale (VI)
R³-SO₂-NH-CO-Z (VI)
dans laquelle
R³ a la signification indiquée ci-dessus, et
Z représente un atome de chlore, un groupe alcoxy en C₁-C₄, un groupe benzyloxy ou un groupe phénoxy,
éventuellement en présence d'un agent neutralisateur d'acides et éventuellement en présence d'un diluant, et, le cas échéant, on forme des sels à partir des composés de formule (I) préparés conformément aux procédés (a), (b) ou (c), conformément à des procédés habituels.

2. Agents herbicides caractérisés par une teneur en au moins une sulfonylaminocarbonyltriazolinone de formule (I) selon la revendication 1.

3. Utilisation de sulfonylaminocarbonyltriazolinones répondant à la formule générale (I) selon la revendication 1, pour lutter contre la croissance non désirée de plantes.

4. Procédé pour lutter contre les mauvaises herbes, caractérisé en ce qu'on laisse agir des sulfonylaminocarbonyltriazolinones répondant à la formule générale (I) selon la revendication 1, sur les mauvaises herbes ou sur leur biotope.

5. Procédé pour la préparation d'agents herbicides, caractérisé en ce qu'on mélange des sulfonylaminocarbonyltriazolinones répondant à la formule générale (I) selon la revendication 1, avec des diluants et/ou des agents tensioactifs.
